# FASCICULE DE BREVET EUROPEEN

(11) **EP 0 662 471 B1**
(45) Date de publication et mention de la délivrance du brevet: **11.03.1998**
(21) Numéro de dépôt: 94402789.5
(22) Date de dépôt: 06.12.1994
(51) Int. Cl.: C07C 233/31, C07C 233/61, C07C 233/18, C07C 233/60, C07C 327/42, C07C 327/46, C07C 275/24, C07C 275/26, A61K 31/16, A61K 31/17, A61K 31/18

(54) **Dérivés naphtaléniques ayant une affinité pour les récepteurs de la mélatonine, leur procédé de préparation et les compositions pharmaceutiques qui les contiennent**
Naphthalen-Derivate mit Affinität für Melatonin-Rezeptoren, Verfahren zu ihrer Herstellung und sie enthaltende pharmazeutische Zusammensetzungen
Naphthalene derivatives with affinity to the receptors of melatonine, process for their preparation and pharmaceutical compositions containing them

(30) Priorité: 07.12.1993 FR 9314630
(43) Date de publication de la demande: 12.07.1995
(73) Titulaire: ADIR ET COMPAGNIE, 92415 Courbevoie Cédex (FR)
(72) Inventeur: Depreux, Patrick, F-59280 Armentieres (FR); Hait Mansour, Hamid, F-59100 Roubaix (FR); Lesieur, Daniel, F-59147 Gondecourt (FR); Lefoulon, François, F-45000 Orleans (FR); Renard, Pierre, F-78000 Versailles (FR); Adam, Gérard, F-78600 Le Mesnil Le Roi (FR); Delagrange, Philippe, F-92130 Issy Les Moulineaux (FR)

(56) Documents cités:
- EP-A- 0 447 285
- EP-A- 0 530 087
- EP-A- 0 562 956
- WO-A-89/01472
- JOURNAL OF MEDICINAL CHEMISTRY, vol.35, no.8, 1992, WASHINGTON US pages 1484 - 1486 S. YOUS ET AL. 'Novel naphthalenic ligands with high affinity for the melatonin receptor'

## Description

La présente invention concerne de nouveaux dérivés à structure naphtalénique, leur procédé de préparation, et les compositions pharmaceutiques qui les contiennent.

Le brevet EP 447285 décrit des dérivés naphtaléniques monosubstitués sur le cycle portant la chaîne alkylamide. La demande de brevet EP 530087 décrit des dérivés naphtylalkyluréiques et naphtylalkylthiouréiques également monosubstitués sur le cycle portant la chaîne alkylurée. Ces composés possèdent de nombreuses et intéressantes activités pharmacologiques de par leur affinité pour les récepteurs de la mélatonine. La demande de brevet EP 562956 décrit des dérivés naphtaléniques monosubstitués sur le cycle portant la chaîne alkylamide qui présentent un caractère antagoniste par rapport à la mélatonine. Le document DE-388566 décrit le 1-acétyl-4-(2-acétylamino-éthyl)-naphtalène mais uniquement en tant qu'intermédiaire de synthèse.

Outre leur action bénéfique sur les troubles du rythme circadien (J. Neurosurg (63), sept. 1985, page 333) et du sommeil (Psychopharmacology, 1990, 100, page 222), les composés agissant sur le système mélatoninergique possèdent d'intéressantes propriétés pharmacologiques sur le système nerveux central, notamment anxiolytiques (Neuropharmacology of Pineal Secretions, vol. 8, N° 3-4, 1990, page 272), antipsychotiques (Neuropharmacology of Pineal Secretions, vol 8, n°3-4, 1990, page 267), analgésiques (Pharmacopsychiat., 20, 1987, page 222), pour le traitement de la maladie de Parkinson (J.Neurosurg (63), sept. 1985, page 331) et d'Alzheimer (Brain Research, 528, 1990, page 173). De même, ces composés ont montré une activité sur certains cancers (Melatonin - clinical Perspectives, 1988, page 164-165), sur l'ovulation (Science vol 227, page 719-720), l'immunomodulation (Adv. Pineal Research, vol. 5, 1991) et le diabète (Clinical endocrinology, 24, 1986, page 363).

La demanderesse a découvert de nouveaux dérivés naphtaléniques disubstitués sur le cycle portant la chaîne alkylamide qui sont de puissants ligands des récepteurs de la mélatonine.

Plus particulièrement, l'invention concerne les composés de formule (I) : 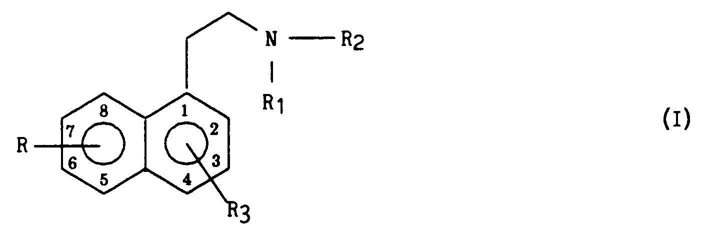 dans laquelle :
- R représente un hydrogène ou un radical choisi parmi alkyle, alkyle substitué et -O-R' ; avec R' représentant un hydrogène ou un radical alkyle, alkyle substitué, cycloalkyle, cycloalkyle substitué, cycloalkylalkyle, cycloalkylalkyle substitué, phényle, phényle substitué, phénylalkyle, phénylalkyle substitué, diphénylalkyle, diphénylalkyle substitué ;
- R₁ représente un hydrogène ou un alkyle ;
- R₂ signifie
   a) 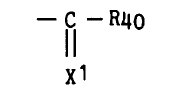 dans lequel X¹ représente un atome de soufre ou d'oxygène et R₄₀ représente un hydrogène ou un radical choisi parmi alkyle, alkyle substitué, cycloalkyle, cycloalkyle substitué, cycloalkylalkyle, cycloalkylalkyle substitué, alkényle, alkényle substitué, alkynyle et alkynyle substitué ; ou
   b) 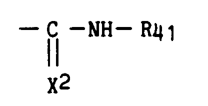 dans laquelle X² représente un soufre ou un oxygène et R₄₁ représente un hydrogène ou un radical choisi parmi alkyle, cycloalkyle et cycloalkylalkyle ;
- R₃ représente un radical choisi parmi :
   - (C₂-C₆) alkyle,
   - (C₂-C₆) alkyle substitué,
   - cycloalkylalkyle,
   - cycloalkylalkyle substitué,
   - alkényle,
   - alkényle substitué,
   - alkynyle,
   - alkynyle substitué,
   - hydroxyle en position 3,
   - R₅-alkyle-, avec R₅ étant substitué ou non substitué et représentant un radical choisi parmi pyridyle, phényle, naphtyle, thiényle, furyle, pyrimidyle, indolyle, benzofuryle, benzothiényle, quinolyle et isoquinolyle ;
   - R₆-CO-O- ,
   - et R₆-CO- ,
      avec R₆ représentant un radical choisi parmi (C₁-C₅)alkyle, (C₁-C₅)alkyle substitué, cycloalkyle, cycloalkyle substitué, cycloalkyl(C₁-C₅)alkyle, cycloalkyl(C₁-C₅)alkyle substitué, R₅- et R₅-alkyle- où R₅ étant non substitué ou substitué est tel que défini précédemment,
      étant entendu que le composé de formule générale (I) ne peut pas être le N-{2-[(4-acétyl)napht-1-yl]éthyl}acétamide,
   - les termes "alkyle" et "alkoxy" désignant, sauf mention contraire, des groupements linéaires ou ramifiés de 1 à 6 atomes de carbone,
   - les termes "alkényle" et "alkynyle" désignant des groupements insaturés, linéaires ou ramifiés, de 2 à 6 atomes de carbone,
   - le terme "cycloalkyle" désignant un groupement cyclique de 3 à 8 atomes de carbone,
   - le terme "substitué" affecté à "alkyle", "alkoxy", "alkényle", "alkynyle", "cycloalkyle" ou "cycloalkylalkyle" signifie que ces groupements sont substitués par un ou plusieurs radicaux choisi parmi alkyle, alkoxy et halogène ;
   - le terme "substitué" associé à R₅ et à "phényle", "phénylalkyle" ou "diphénylalkyle" signifie que ces groupements sont substitués par un ou plusieurs radicaux choisis parmi alkyle, alkoxy, halogène, hydroxy et trifluorométhyle ;
   - leurs énantiomères et diastéreoisomères,
   - et leurs sels d'addition à un acide ou à une base pharmaceutiquement acceptable.

L'invention concerne par exemple les composés de formule (I) dans laquelle :
- R₃ est attaché en position 3 du noyau naphtalène
- R₃ est un hydroxyle,
- R₃ est un (C₂-C₆)alkyle,
- R₃ est un cycloalkylalkyle,
- R₃ est un groupement R₅-alkyle-,
- R₃ est un groupement R₅-alkyle- dans lequel R₅ est un phényle,
- R₃ est un groupement R₆-CO-O-,
- R₃ est un groupement R₆-CO-,
- R₆ est un (C₁-C₅) alkyle,
- R₆ est un cycloalkyle,
- R₆ est un cycloalkylalkyle,
- R₆ est un phényle,
- R est attaché en position 7 du noyau naphtalène,
- R est un hydrogène,
- R est un hydroxyle,
- R est un alkyle,
- R est un alkoxy,
- R₁ est un hydrogène,
- R₂ est un groupement -CO-R₄₀
- R₂ est un groupement -CS-R₄₀,
- R₄₀ est un alkyle,
- R₄₀ est un cycloalkyle,
- R₄₀ est un alkényle,
- R₂ est un groupement -CX-NH-R₄₁,
- X est un oxygène,
- X est un soufre,
- R₄₁ est un alkyle,
- ou R₄₁ est un cycloalkyle.

De façon préférentielle, l'invention concerne les composés suivants :
- N-[2-(7-méthoxy 3-benzoyl napht-1-yl)éthyl]acétamide,
- N-[2-(7-méthoxy 3-cyclopropylcarbonyl napht-1-yl)éthyl]acétamide,
- N-[2-(7-méthoxy 3-propionyl napht-1-yl)éthyl]acétamide,
- N-[2-(7-méthoxy 3-acétyl napht-1-yl)éthyl]acétamide,
- N-[2-(7-méthoxy 3-acétyl napht-1-yl)éthyl]cyclopropylcarboxamide,
- N-[2-(7-méthoxy 3-hydroxy napht-1-yl)éthyl]acétamide,
- N-[2-(3,7-dihydroxy napht-1-yl)éthyl]carboxamide,
- N-[2-(7-méthoxy 3-benzyl napht-1-yl)éthyl]acétamide,
- N-[2-(7-méthoxy 3-éthyl napht-1-yl)éthyl]acétamide,
- N-[2-(7-méthoxy 3-cyclopropylméthyl napht-1-yl)éthyl]acétamide,
- N-[2-(7-méthoxy 3-propyl napht-1-yl)éthyl]acétamide,
- N-[2-(7-méthoxy 3-éthyl napht-1-yl)éthyl]cyclopropylcarboxamide.

Parmi les acides pharmaceutiquement acceptables que l'on peut utiliser pour former un sel d'addition avec les composés de l'invention, on peut citer à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

De la même façon, parmi les bases pharmaceutiquement acceptables que l'on peut utiliser pour former un sel d'addition, on peut citer les hydroxydes de sodium, de potassium, de calcium, ou d'aluminium, les carbonates de métaux alcalins ou alcalinoterreux, et les bases organiques comme la triéthylamine, la benzylamine, la diéthanolamine, la tert.butylamine, la dicyclohexylamine, et l'arginine.

De façon particulière, les radicaux alkyles présents dans la formule (I) peuvent être choisis parmi méthyle, éthyle, n-propyle, isopropyle, n-butyle, isobutyle, sec-butyle, tert-butyle, pentyle, ou hexyle.

Les radicaux alkoxy présents dans la formule (I) peuvent être choisis parmi méthoxy, éthoxy, n-propoxy, isopropoxy, n-butoxy, iso-butoxy, tert-butoxy, pentyloxy, et hexyloxy.

Les halogènes présents dans la formule (I) peuvent être choisis parmi le chlore, le fluor, le brome et l'iode. Les radicaux cycloalkyles présents dans la formule (I) peuvent être choisis parmi cyclopropyle, cyclobutyle, cyclopentyle, cyclohexyle, cycloheptyle et cyclooctyle.

L'invention s'étend également au procédé de préparation des composés de formule (I), dans lequel on fait réagir un composé de formule (II) : 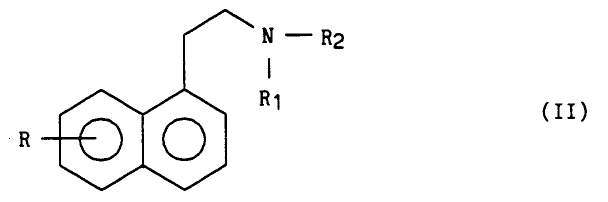 dans laquelle R, R₁, et R₂ sont tels que définis dans la formule (I), avec un composé de formule (III) :

R₆-COCl (III)

dans laquelle R₆ est tel que défini dans la formule (I), pour obtenir un composé de formule (Ia) correspondant : 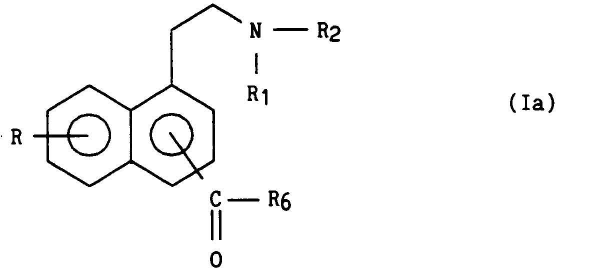 dans laquelle R, R₁, R₂ et R₆ sont tels que définis précédemment, qui peut être, si on le désire,
- soit soumis à une oxydation par une réaction de Baeyer-Villiger, pour obtenir un composé de formule (Ib) correspondant : 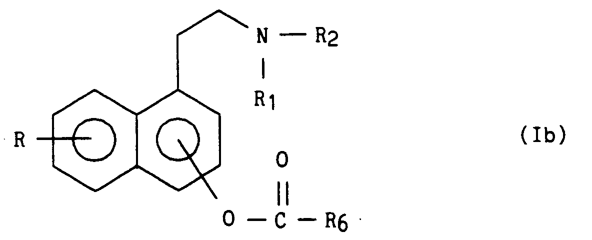 dans laquelle R, R₁, R₂ et R₆ sont tels que définis précédemment, puis si le substituant -O-CO-R₆ est en position 3, soumis à une saponification en présence d'hydroxyde de sodium, pour obtenir un composé de formule (Ic) correspondant : 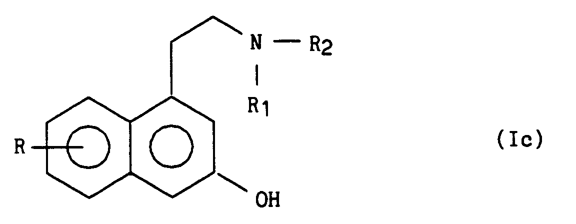 dans laquelle R, R₁, R₂ sont tels que définis précédemment,
- soit soumis à une réduction par le mercure et le zinc, en présence de toluène et d'acide chlorhydrique, pour obtenir un composé de formule (Id) correspondant : 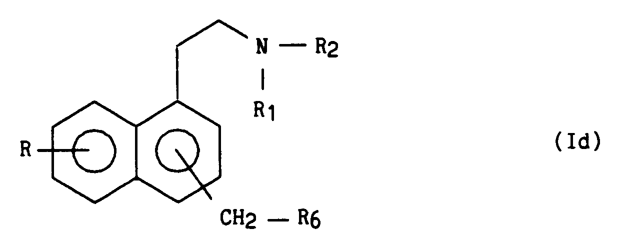 dans laquelle R, R₁, R₂ et R₆ sont tels que définis précédemment,
les composés de formule (Ia), (Ib), (Ic), et (Id) formant l'ensemble des composés de formule (I), composés de formule (I) qui peuvent être,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou une base pharmaceutiquement acceptable.

Plus particulièrement, l'invention concerne le procédé de préparation des composés de formule (I'), cas particulier des composés de formule (I) dans laquelle R₃ est en position 3 du naphtalène, dans lequel on fait réagir un composé de formule (II) : 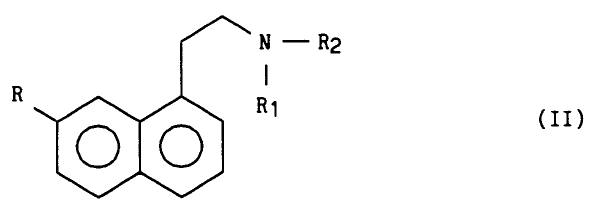 dans laquelle R, R₁, et R₂ sont tels que définis dans la formule (I), avec un composé de formule (III) :

R₆-COCl (III)

dans laquelle R₆ est tel que défini dans la formule (I), pour obtenir un composé de formule (Ia') correspondant : 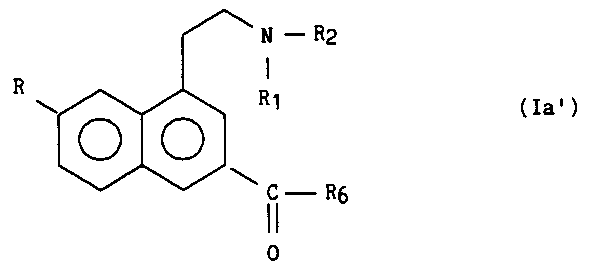 dans laquelle R, R₁, R₂ et R₆ sont tels que définis précédemment, qui peut être, si on le désire,
- soit soumis à une oxydation par une réaction de Baeyer-Villiger, pour obtenir un composé de formule générale (Ib') correspondant : 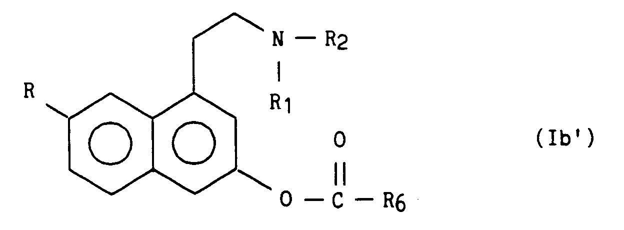 dans laquelle R, R₁, R₂ et R₆ sont tels que définis précédemment, puis éventuellement soumis à une saponification en présence d'hydroxyde de sodium, pour obtenir un composé de formule (Ic') correspondant : 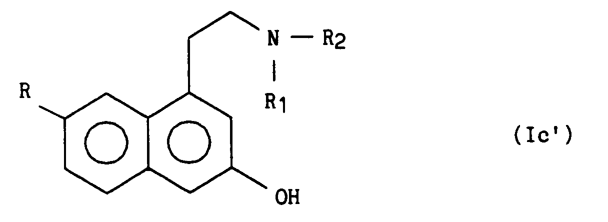 dans laquelle R, R₁, R₂ sont tels que définis précédemment,
- soit soumis à une réduction par le mercure et le zinc, en présence de toluène et d'acide chlorhydrique, pour obtenir un composé de formule (Id') correspondant : 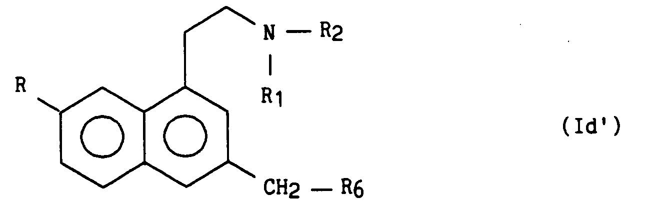 dans laquelle R, R₁, R₂ et R₆ sont tels que définis précédemment,
   les composés de formule (Ia'), (Ib'), (Ic'), et (Id') formant l'ensemble des composés de formule (I'),
   composés de formule (I') qui peuvent être si on le désire,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou une base pharmaceutiquement acceptable.

Les composés de formule (Ia), (Ib), (Ic), (Id) peuvent également être soumis si R représente -OCH₃, à une réaction de déméthylation par le tribromure de bore. Plus particulièrement les composés de formule (Ia'), (Ib'), (Ic'), (Id') peuvent être soumis, si R représente -OCH₃, à une réaction de déméthylation par le tribromure de bore. Les composés hydroxylés correspondants obtenus font également partis des composés de formule (I) selon l'invention.

Les matières premières utilisées dans le procédé précédemment décrit sont soit commerciales, soit aisément accessibles à l'homme du métier selon des procédés bien connus dans la littérature. On se référera plus particulièrement, pour les composés de formule générale (II), aux descriptions du brevet EP 447285 et de la demande de brevet EP 530087.

Les composés thiocarboxamides sont aisément obtenus par l'homme du métier, notamment par utilisation du réactif de Lawesson.

Les composés de formule (I) possèdent des propriétés pharmacologiques très intéressantes et sont utiles pour le traitement des troubles mélatoninergiques.

L'étude pharmacologique des dérivés de l'invention a en effet montré qu'ils n'étaient pas toxiques, doués d'une très haute affinité sélective pour les récepteurs de la mélatonine et d'importantes activités sur le système nerveux central et en particulier, on a relevé des propriétés bénéfiques sur les troubles du sommeil, des propriétés anxiolytiques, antipsychotiques, analgésiques ainsi que des propriétés avantageuses sur la microcirculation qui permettent d'établir que les produits de l'invention sont utiles dans le traitement du stress, des troubles du sommeil, de l'anxiété, des dépressions saisonnières, des insomnies et fatigues dues aux décalages horaires, de la schizophrénie, des attaques de panique, de la mélancolie, de la régulation de l'appétit, des troubles psychotiques, de l'épilepsie, de la maladie de Parkinson, de la démence sénile, des divers désordres liés au vieillissement normal ou pathologique, de la migraine, des pertes de mémoire, de la maladie d'Alzheimer, ainsi que les troubles de la circulation cérébrale. Dans un autre domaine d'activité, il apparaît que les produits de l'invention possèdent des propriétés d'inhibiteurs de l'ovulation, d'immunomodulateurs et qu'ils sont susceptibles d'être utilisés dans le traitement de certains cancers hormonodépendants.

Les composés seront utilisés de préférence dans les traitements des dépressions saisonnières, des troubles du sommeil, des pathologies cardiovasculaires, des insomnies et fatigues dues aux décalages horaires, et des troubles de l'appétit et de l'obésité.

Par exemple, les composés seront utilisés dans le traitement des dépressions saisonnières et des troubles du sommeil.

Les composés de l'invention possèdent également un profil métabolique très avantageux.

La présente invention a également pour objet les compositions pharmaceutiques contenant les produits de formule (I) ou le cas échéant un de leurs sels d'addition à un acide ou une base pharmaceutiquement acceptable en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon l'âge et le poids du patient, la voie d'administration, la nature de l'indication thérapeutique, ou des traitement éventuellement associés et s'échelonne entre 0,1 mg et 1 g par 24 heures en 1 ou 2 prises, plus particulièrement 1 à 100 mg, par exemple 1 à 10 mg.

Les exemples suivants illustrent l'invention, mais ne la limitent en aucune façon.

### EXEMPLE 1 : N-[2-(7-METHOXY 3-BENZOYL NAPHT-1-YL)ETHYL]ACETAMIDE

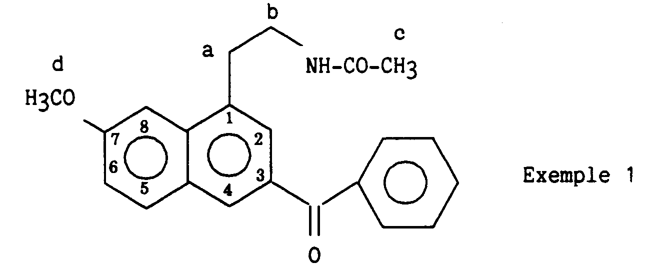

A une solution de 11 g (45,2 mmole) de N-[2-(7-méthoxy napht-1-yl)éthyl]acétamide et de 7,7 g (57,7 mmole) de AlCl₃, dans 60 cm³ de nitrobenzène, ajouter goutte à goutte 57,9 mmole de chlorure d'acide benzoïque, à 12°C et sous azote. Laisser agir pendant 2 heures à 12°C et verser le mélange réactionnel sur la glace. Extraire avec le dichlorométhane, sécher sur le sulfate de magnésium, concentrer puis chromatographier le brut sur silice (éluant CH₂Cl₂/CH₃OH: 99/1).

On obtient le composé de l'exemple 1 :
- Rendement : 34 %
- Point de fusion : 92°-95°C
- Solvant de recristallisation : Toluène/Hexane
- Masse moléculaire : 365, 411 pour C₂₂H₂₁NO₃ ; 1H₂O
- Microanalyse :

| | **C %** | **H %** |
|---|---|---|
| Calculé | 72,30 | 6,34 |
| Trouvé | 72,55 | 6,30 |

- Infra-Rouge :
ν N-H (amide) : 3340 cm⁻¹
ν C = O (cétonique) : 1660 cm⁻¹
ν C = O (amide) : 1625 cm⁻¹

- RMN (DMSO,d6) 300 MHZ
1,84 ppm (singulet, 3H, (Hc))
3,23 ppm (triplet, 2H, (Ha))
3,37 ppm (quadruplet, 2H, (Hb))
4,00 ppm (singulet, 3H, (Hd))
7,28 ppm (doublet, 1H, (H₆), J₆₋₅ = 9Hz)
7,57-7,81 ppm (massif, 7H, [H₂, H₄, H(A)])
8,00 ppm (doublet, 1H,(H₅), J₅₋₆ = 9 Hz)
8,14 ppm (multiplet, 2H, (H₈, NH))

### EXEMPLES 2 A 15 :

En procédant comme dans l'exemple 1 mais en remplaçant le N-[2-(7-méthoxy napht-1-yl)éthyl] acétamide par le composé convenablement substitué sur la fonction amide, on obtient les composés des exemples suivants :
- **EXEMPLE 2 :**: **N-[2-(7-METHOXY 3-BENZOYL NAPHT-1-YL)ETHYL]PROPIONAMIDE**
- **EXEMPLE 3 :**: **N-[2-(7-METHOXY 3-BENZOYL NAPHT-1-YL)ETHYL]BUTYRAMIDE**
- **EXEMPLE 4 :**: **N-[2-(7-METHOXY 3-BENZOYL NAPHT-1-YL)ETHYL]PENTANAMIDE**
- **EXEMPLE 5 :**: **N-[2-(7-METHOXY 3-BENZOYL NAPHT-1-YL)ETHYL]HEXANAMIDE**
- **EXEMPLE 6 :**: **N-[2-(7-METHOXY 3-BENZOYL NAPHT-1-YL)ETHYL]** **CYCLOPROPYLCARBOXAMIDE**
- **EXEMPLE 7 :**: **N-[2-(7-METHOXY 3-BENZOYL NAPHT-1-YL)ETHYL]** **CYCLOBUTYLCARBOXAMIDE**
- **EXEMPLE 8 :**: **N-[2-(7-METHOXY 3-BENZOYL NAPHT-1-YL)ETHYL]** **CYCLOPENTYLCARBOXAMIDE**
- **EXEMPLE 9 :**: **N-[2-(7-METHOXY 3-BENZOYL NAPHT-1-YL)ETHYL]** **CYCLOHEXYLCARBOXAMIDE**
- **EXEMPLE 10 :**: **N-[2-(7-METHOXY 3-BENZOYL NAPHT-1-YL)ETHYL] N'-METHYLUREE**
- **EXEMPLE 11 :**: **N-[2-(7-METHOXY 3-BENZOYL NAPHT-1-YL)ETHYL] N'-ETHYLUREE**
- **EXEMPLE 12 :**: **N-[2-(7-METHOXY 3-BENZOYL NAPHT-1-YL)ETHYL] N'-PROPYLUREE**
- **EXEMPLE 13 :**: **N-[2-(7-METHOXY 3-BENZOYL NAPHT-1-YL)ETHYL] N'-HEXYLUREE**
- **EXEMPLE 14 :**: **N-[2-(7-METHOXY 3-BENZOYL NAPHT-1-YL)ETHYL] N'-CYCLOPROPYLUREE**
- **EXEMPLE 15 :**: **N-[2-(7-METHOXY 3-BENZOYL NAPHT-1-YL)ETHYL] N'-CYCLOHEXYLUREE**

### EXEMPLE 16 A 26 :

En procédant comme dans l'exemle 1 mais en remplaçant le N-[2(7-méthoxy napht-1-yl)ethyl]acétamide par le composé convenablement substitué en position 7 du naphtalène ainsi que le cas échéant convenablement substitué sur la fonction amide, on obtient les composés des exemples suivants :
- **EXEMPLE 16 :**: **N-[2-(3-BENZOYL NAPHT-1-YL)ETHYL]ACETAMIDE**
- **EXEMPLE 16 bis :**: **N-[2-(7-ETHOXY 3-BENZOYL NAPHT-1-YL)ETHYL]ACETAMIDE**
- **EXEMPLE 17 :**: **N-[2-(7-PROPOXY 3-BENZOYL NAPHT-1-YL)ETHYL]ACETAMIDE**
- **EXEMPLE 18 :**: **N-[2-(7-PENTOXY 3-BENZOYL NAPHT-1-YL)ETHYL]ACETAMIDE**
- **EXEMPLE 19 :**: **N-[2-(7-ALLYLOXY 3-BENZOYL NAPHT-1-YL)ETHYL]ACETAMIDE**
- **EXEMPLE 20 :**: **N-[2-(7-PROPARGYLOXY 3-BENZOYL NAPHT-1-YL)ETHYL]ACETAMIDE**
- **EXEMPLE 21 :**: **N-[2-(7-CYCLOPROPYLETHYLOXY 3-BENZOYL NAPHT-1-YL)ETHYL**]**ACETAMIDE**
- **EXEMPLE 22 :**: **N-[2-(7-CYCLOHEXYLOXY 3-BENZOYL NAPHT-1-YL)ETHYL]ACETAMIDE**
- **EXEMPLE 23 :**: **N-[2-(7-CYCLOHEXEN-2-YLOXY 3-BENZOYL NAPHT-1-YL)ETHYL**]**ACETAMIDE**
- **EXEMPLE 24 :**: **N-[2-(7-BENZYLOXY-3-BENZOYL NAPHT-1-YL)ETHYL]ACETAMIDE**
- **EXEMPLE 25 :**: **N-[2-(3-BENZOYL NAPHT-1-YL)ETHYL]CYCLOPROPYLCARBOXAMIDE**
- **EXEMPLE 26 :**: **N-[2-(3-BENZOYL NAPHT-1-YL)ETHYL]CYCLOBUTYLCARBOXAMIDE**
- **EXEMPLE 27 :**: **N-[2-(7-METHOXY 3-CYCLOPROPYLCARBONYL NAPHT-1-YL)ETHYL]ACETAMIDE**

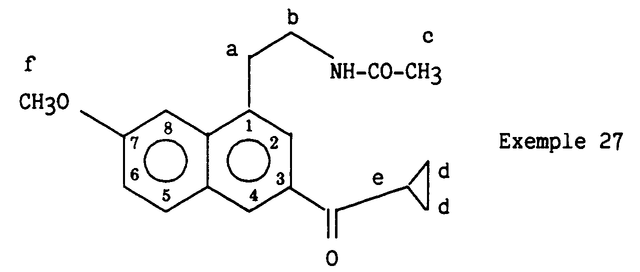

En procédant de la même façon que dans l'exemple 1, mais en utilisant pour la réaction d'acylation du chlorure de l'acide cyclopropane carboxylique, on obtient le composé de l'exemple 27 :
- Rendement :29 %
- Solvant de recristallisation :Cyclohexane
- Point de fusion :152°C
- Masse moléculaire :309, 349 pour C₁₉H₁₉NO₃
- Microanalyse :

| | **C %** | **H %** | **N %** |
|---|---|---|---|
| Calculé | 73,28 | 6,79 | 4,49 |
| Trouvé | 73,00 | 6,83 | 4,38 |

- Infra-Rouge :
ν N-H (amide) = 3340 cm⁻¹
ν C = O (cétonique) : 1660 cm⁻¹
ν C = O (amide) : 1630 cm⁻¹

- RMN (DMSO,d6)
1,05 ppm (doublet, 4H, (H_{d}))
1,80 ppm (singulet, 3H, (H_{c}))
3,05 ppm (triplet, 1H, (Hₑ))
3,20 ppm (triplet, 2H, (Hₐ))
3,35 ppm (multiplet, 2H, (H_{b}))
4,00 ppm (singulet, 3H, (H_{f}))
7,30 ppm (doublet, 1H, (H₆) J₅₋₆ = 8,80 Hz)
7,70 ppm (singulet, 1H, (H₈))
7,85 ppm (singulet, 1H, (H₂ ou H₄))
8,05 ppm (doublet, 1H, (H₅) J₅₋₆ = 8,80 Hz)
8,13 ppm (signal, 1H, NH)
8,65 ppm (singulet, 1H (H₄ ou H₂))

### EXEMPLES 28 A 33 :

En procédant comme dans l'exemple 27 mais en remplaçant le N-[2-(7-méthoxy napht-1-yl)éthyl]acétamide par le composé convenablement substitué sur la fonction amide, on obtient les composés des exemples suivants :
- **EXEMPLE 28 :**: **N-[2-(7-METHOXY 3-CYCLOPROPYLCARBONYL NAPHT-1-YL)ETHYL**]**PROPIONAMIDE**
- **EXEMPLE 29 :**: **N-[2-(7-METHOXY 3-CYCLOPROPYLCARBONYL NAPHT-1-YL)ETHYL]BUTYRAMIDE**
- **EXEMPLE 30 :**: **N-[2-(7-METHOXY 3-CYCLOPROPYLCARBONYL NAPHT-1-YL)ETHYL]CYCLOPROPYL CARBOXAMIDE**
- **EXEMPLE 31 :**: **N-[2-(7-METHOXY 3-CYCLOPROPYLCARBONYL NAPHT-1-YL)ETHYL**]**CYCLOBUTYL CARBOXAMIDE**
- **EXEMPLE 32 :**: **N-[2-(7-METHOXY 3-CYCLOPROPYLCARBONYL NAPHT-1-YL)ETHYL]** **N'-METHYL UREE**
- **EXEMPLE 33 :**: **N-[2-(7-METHOXY 3-CYCLOPROPYLCARBONYL NAPHT-1-YL)ETHYL]** **N'-PROPYL UREE**

### EXEMPLES 34 A 37 :

En procédant comme dans l'exemple 27 mais en remplaçant le N-[2-(7-méthoxy napht-1-yl)éthyl]acétamide pour le composé convenablement substitué en position 7 du naphtalène et, le cas échéant convenablement substitué sur la fonction amide, on obtient les composés des exemples suivants :
- **EXEMPLES 34 :**: **N-[2-(3-CYCLOPROPYLCARBONYL NAPHT-1-YL)ETHYL]ACETAMIDE**
- **EXEMPLES 35 :**: **N-[2-(7-CYCLOPROPYLMETHYLOXY 3-CYCLOPROPYL CARBONYL)ETHYL**]**ACETAMIDE**
- **EXEMPLES 36 :**: **N-[2-(3-CYCLOPROPYLCARBONYL NAPHT-1-YL)ETHYL**]**CYCLOPROPYLCARBOXAMIDE**
- **EXEMPLES 37 :**: **N-[2-(3-CYCLOPROPYLCARBONYL NAPHT-1-YL)ETHYL]CYCLOBUTYLCARBOXAMIDE**
- **EXEMPLE 38 :**: **N-[2-(7-METHOXY 3-PROPIONYL NAPHT-1-YL)ETHYL]ACETAMIDE**

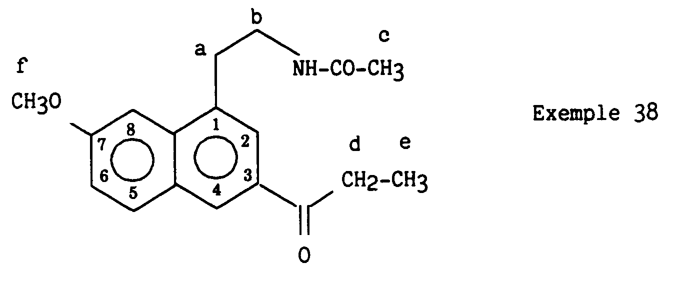

En procédant de la même façon que dans l'exemple 1, mais en utilisant pour la réaction d'acylation le chlorure de propionyle, on obtient le composé de l'exemple 38 :
- Rendement : 47 %
- Solvant de recristallisation : Toluène
- Point de fusion : 141°-143°C
- Masse moléculaire : 299, 255 pour C₁₈H₂₁NO₃
- Microanalyse :

| | **C %** | **H %** | **N %** |
|---|---|---|---|
| Calculé | 72,21 | 7,07 | 4,68 |
| Trouvé | 72,51 | 6,99 | 4,52 |

- Infra-Rouge :
νN-H (amide) = 3380 cm⁻¹
ν C = O (cétonique) = 1665 cm⁻¹
ν C = O (amide) = 1610 cm⁻¹

- RMN (DMSO,d6)
1,13 ppm (triplet, 3H, (Hₑ))
1,83 ppm (singulet, 3H, (H_{c}))
3,15 ppm (multiplet, 4H, (Hₐ, H_{d}))
3,32 ppm (multiplet, 2H, (H_{b}))
4,00 ppm (singulet,3H, (H))
7,30 ppm (doublet dédoublé, 1H, (H₆) J₆₋₅ = 9,00 Hz, J₆₋₈ = 2,25 Hz)
7,70 ppm (doublet, 1H, (H₈) J₂₋₆ = 2,25 Hz)
7,80 ppm (singulet, 1H, (H₂ ou H₄))
8,00 ppm (doublet, 1H, (H₅) J₅₋₆ = 9,00 Hz)
8,15 ppm (triplet, 1H, (NH))
8,50 ppm (singulet, 1H, (H₄ ou H₂))

### EXEMPLES 39 A 44 :

En procédant comme dans l'exemple 38 mais en remplaçant le N-[2(7-méthoxy napht-1-yl)éthyl] acétamide par le composé convenablement substitué sur la fonction amide, on obtient les composés des exemples suivants :
- **EXEMPLE 39 :**: **N-[2-(7-METHOXY 3-PROPIONYL NAPHT-1-YL)ETHYL]PROPIONAMIDE**
- **EXEMPLE 40 :**: **N-[2-(7-METHOXY 3-PROPIONYL NAPHT-1-YL)ETHYL]BUTYRAMIDE**
- **EXEMPLE 41 :**: **N-[2-(7-METHOXY 3-PROPIONYL NAPHT-1-YL)ETHYL]CYCLOPROPYLCARBOXAMIDE**
- **EXEMPLE 42 :**: **N-[2-(7-METHOXY 3-PROPIONYL NAPHT-1-YL)ETHYL]CYCLOBUTYLCARBOXAMIDE**
- **EXEMPLE 43 :**: **N-[2-(7-METHOXY 3-PROPIONYL NAPHT-1-YL)ETHYL]N'-METHYL UREE**
- **EXEMPLE 44 :**: **N-[2-(7-METHOXY 3-PROPIONYL NAPHT-1-YL)ETHYL]N'-PROPYL UREE**

### EXEMPLES 45 à 48 :

En procédant comme dans l'exemple 38 mais en remplacant le N-[2-(7-méthoxy napht-1-yl)éthyl]acétamide par le composé convenablement substitué en position 7 du naphtalène et, le cas échéant convenablement substitué sur la fonction amide, on obtient les composés des exemples suivants :
- **EXEMPLE 45 :**: **N-[2-(3-PROPIONYL NAPHT-1-YL)ETHYL]ACETAMIDE**
- **EXEMPLE 46 :**: **N-[2-(7-CYCLOPROPYLMETHYLOXY 3-PROPIONYL NAPHT-1-YL)ETHYL]ACETAMIDE**
- **EXEMPLE 47 :**: **N-[2-(3-PROPIONYL NAPHT-1-YL)ETHYL]CYCLOPROPYLCARBOXAMIDE**
- **EXEMPLE 48 :**: **N-[2-(3-PROPIONYL NAPHT-1-YL)ETHYL]CYCLOBUTYLCARBOXAMIDE**
- **EXEMPLE 49 :**: **N-[2-(7-METHOXY 3-ACETYL NAPHT-1-YL)ETHYL]ACETAMIDE**

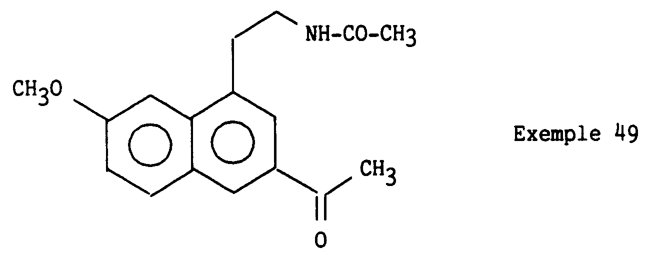

En procédant comme dans l'exemple 1 en utilisant pour la réaction d'acylation le chlorure d'acétyle, on obtient le composé de l'exemple 49 :
- Masse moléculaire : 285,346 pour C₁₇ H₁₉ NO₃
- Point de fusion : 154,5°C
- Microanalyse :

| | **C %** | **H %** | **N %** |
|---|---|---|---|
| Calculé | 71,56 | 6,71 | 4,91 |
| Trouvé | 71,41 | 6,67 | 4,89 |

### EXEMPLE 50 : N-[2-(7-METHOXY 3-ACETYL NAPHT-1-YL)ETHYL] CYCLOPROPYLCARBOXAMIDE

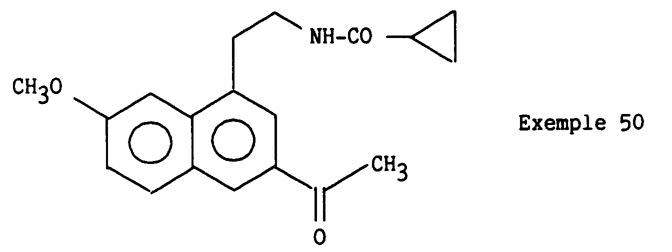

En procédant comme dans l'exemple 1 en utilisant comme matière première le N-[2-(7-méthoxy napht-1-yl)éthyl]cyclopropylcarboxamide et le chlorure d'acétyle, on obtient le composé de l'exemple 50.
- Rendement : 31 %
- Point de fusion : 140°-141°C
- Solvant de recristallisation : Toluène
- Masse moléculaire : 311, 365 pour C₁₉H₂₁NO₃
- Microanalyse :

| | **C %** | **H %** | **N %** |
|---|---|---|---|
| Calculé | 73,28 | 6,79 | 4,49 |
| Trouvé | 73,08 | 6,74 | 4,43 |

- Infra-Rouge :
νN-H (amide) : 3360 cm⁻¹
ν C = O (cétonique) : 1680 cm⁻¹
ν C = O (amide) : 1600 cm⁻¹

- RMN (DMSO,d6)
0,65 ppm (multiplet, 4H, (H_{d}))
2,65 ppm (singulet, 3H, (H_{c}))
3,20 ppm (triplet, 2H, (Hₐ))
3,40 ppm (multiplet, 2H, (H_{b}))
4,00 ppm (singulet,3H, (H_{f}))
7,30 ppm (doublet dédoublé, 1H, (H₆) J₆₋₅ = 8,90 Hz, J₆₋₈ = 2,00 Hz)
7,60 ppm (doublet, 1H, (H₈) J₈₋₆ = 2,00 Hz)
7,80 ppm (singulet, 1H, (H₂ ou H₄))
8,00 ppm (doublet, 1H, (H₅) J5-6 = 8,90 HZ)
8,30 ppm (triplet, 1H, (NH))

### EXEMPLES 51 À 63

En procédant comme dans l'exemple 49 mais en remplaçant le N-[2-(7-méthoxy napht-1-yl)ethyl]acétamide par le composé convenablement substitué sur la fonction amide, on obtient les composés des exemples suivants :
- **EXEMPLE 51 :**: **N-[2-(7-METHOXY 3-ACETYL NAPHT-1-YL)ETHYL PROPIONAMIDE**
- **EXEMPLE 52 :**: **N-[2-(7-METHOXY 3-ACETYL NAPHT-1-YL)ETHYL]BUTYRAMIDE**
- **EXEMPLE 53 :**: **N-[2-(7-METHOXY 3-ACETYL NAPHT-1-YL)ETHYL]PENTANAMIDE**
- **EXEMPLE 54 :**: **N-[2-(7-METHOXY 3-ACETYL NAPHT-1-YL)ETHYL]HEXANAMIDE**
- **EXEMPLE 55 :**: **N-[2-(7-METHOXY 3-ACETYL NAPHT-1-YL)ETHYL**]**CYCLOBUTYLCARBOXAMIDE**
- **EXEMPLE 56 :**: **N-[2-(7-METHOXY 3-ACETYL NAPHT-1-YL)ETHYL]CYCLOPENTYLCARBOXAMIDE**
- **EXEMPLE 57 :**: **N-[2-(7-METHOXY 3-ACETYL NAPHT-1-YL)ETHYL**]**CYCLOHEXYLCARBOXAMIDE**
- **EXEMPLE 58 :**: **N-[2-(7-METHOXY 3-ACETYL NAPHT-1-YL)ETHYL] N'-METHYL UREE**
- **EXEMPLE 59 :**: **N-[2-(7-METHOXY 3-ACETYL NAPHT-1-YL)ETHYL] N'-ETHYL UREE**
- **EXEMPLE 60 :**: **N-[2-(7-METHOXY 3-ACETYL NAPHT-1-YL)ETHYL] N'-PROPYL UREE**
- **EXEMPLE 61 :**: **N-[2-(7-METHOXY 3-ACETYL NAPHT-1-YL)ETHYL] N'-HEXYL UREE**
- **EXEMPLE 62 :**: **N-[2-(7-METHOXY 3-ACETYL NAPHT-1-YL)ETHYL] N'-CYCLOPROPYL** **UREE**
- **EXEMPLE 63 :**: **N-[2-(7-METHOXY 3-ACETYL NAPHT-1-YL)ETHYL] N'-CYCLOHEXYL** **UREE**

### EXEMPLE 64 A 75 :

En procédant comme dans l'exemple 49 mais en remplaçant le N-[2-(7-méthoxy napht-1-yl)éthyl]acétamide par le composé convenablement substitué en position 7 du naphtalène ainsi que le cas échéant convenablement substitué sur la fonction amide, on obtient les composés des exemples suivants :
- **EXEMPLE 64 :**: **N-[2-(3-ACETYL NAPHT-1-YL)ETHYL]ACETAMIDE**
- **EXEMPLE 65 :**: **N-[2-(7-PENTOXY 3-ACETYL NAPHT-1-YL)ETHYL]ACETAMIDE**
- **EXEMPLE 66 :**: **N-[2-(7-PROPOXY 3-ACETYL NAPHT-1-YL)ETHYL]ACETAMIDE**
- **EXEMPLE 67 :**: **N-[2-(7-PENTOXY 3-ACETYL NAPHT-1-YL)ETHYL]ACETAMIDE**
- **EXEMPLE 68 :**: **N-[2-(7-ALLYLOXY 3-ACETYL NAPHT-1-YL)ETHYL]ACETAMIDE**
- **EXEMPLE 69 :**: **N-[2-(7-PROPARGYLOXY 3-ACETYL NAPHT-1-YL)ETHYL]ACETAMIDE**
- **EXEMPLE 70 :**: **N-[2-(7-CYCLOPROPYLMETHYLOXY 3-ACETYL NAPHT-1-YL)ETHYL]ACETAMIDE**
- **EXEMPLE 71 :**: **N-[2-(7-CYCLOHEXYLOXY 3-ACETYL NAPHT-1-YL)ETHYL]ACETAMIDE**
- **EXEMPLE 72 :**: **N-[2-(7-CYCLOHEXEN-2-YLOXY 3-ACETYL NAPHT-1-YL)ETHYL]ACETAMIDE**
- **EXEMPLE 73 :**: **N-[2-(7-BENZYLOXY 3-ACETYL NAPHT-1-YL)ETHYL]ACETAMIDE**
- **EXEMPLE 74 :**: **N-[2-(3-ACETYL NAPHT-1-YL)ETHYL]CYCLOPROPYLCARBOXAMIDE**
- **EXEMPLE 75 :**: **N-[2-(3-ACETYL NAPHT-1-YL)ETHYL]CYCLOBUTYLCARBOXAMIDE**
- **EXEMPLE 76 :**: **N-[2-(7-METHOXY 3-ACETOXY NAPHT-1-YL)ETHYL]ACETAMIDE**

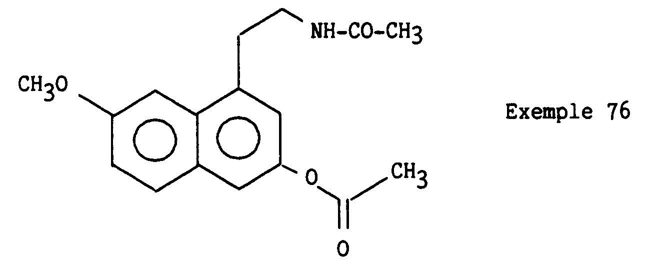

A une solution de 2,8 g (9,81 mmol) de N-[2-(7-méthoxy 3-acétyl napht-1-yl)éthyl]acétamide, selon l'exemple 49, dans 125 cm³ de méthanol, est ajoutée une solution de 7,6 g (12,3 mmol) de sel de magnésium de l'acide monoperoxyphtalique dans 100 cm³ d'eau ajustée à pH 5 avec NaOH 1N. Agiter à température ambiante pendant 24 heures. Evaporer le méthanol, ajouter NaHCO₃ 1N, extraire avec CH₂Cl₂, sécher sur MgSO₄, filtrer et concentrer. On obtient le composé de l'exemple 76.

### EXEMPLE 77 : N-[2-(7-METHOXY 3-HYDROXY NAPHT-1-YL)ETHYL]ACETAMIDE

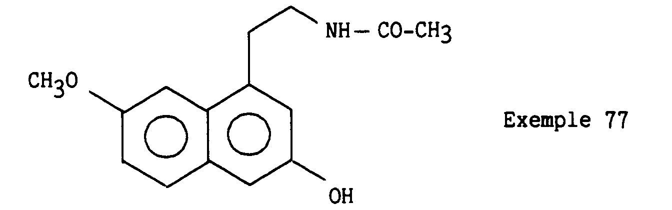

Dissoudre le résidu obtenu dans l'exemple 76, c'est à dire le N-[2-(7-méthoxy 3-acétoxy napht-1-yl)éthyl]acétamide, dans 200 cm³ de méthanol et traiter avec 250 cm³ de NaOH 0,05 N durant 1 heure à température ambiante. Evaporer le MeOH, amener à pH 12 avec NaOH N, extraire avec CH₂Cl₂, sécher sur MgSO₄, filtrer et concentrer. Chromatographier sur silice éluant avec CH₂Cl₂/MeOH = 98/2. On obtient 2,3 g (35 %) du composé de l'exemple 77.
- Masse moléculaire : 259,307
- Point de fusion : 154,8°C
- solvant de cristallisation : CH₂Cl₂
- Microanalyse :

| | **C %** | **H %** | **N %** |
|---|---|---|---|
| Calculé | 69,48 | 6,61 | 5,40 |
| Trouvé | 68,75 | 6,40 | 5,54 |

### EXEMPLE 78 : N-[2-(3,7-DIHYDROXY NAPHT-1-YL)ETHYL]ACETAMIDE

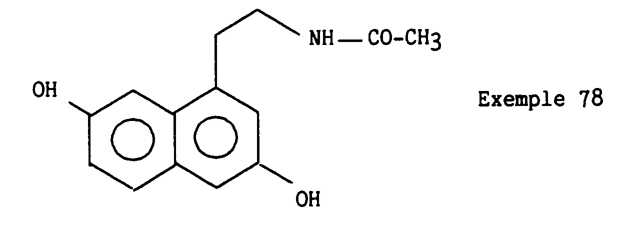

Dissoudre 0,2 g (0,771 mmol) de N-[2-(7-méthoxy 3-hydroxy napht-1-yl)]acétamide selon l'exemple 77 dans 5 cm³ de dichlorométhane anhydre. Refroidir dans un bain de glace et sel. Ajouter goutte à goutte 0,8 cm³ de tribromure de bore 1M. Agiter dans la glace à température ambiante pendant 4 heures. Ajouter à nouveau 0,8 cm³ de tribromure de bore 1M dans CH₂Cl₂. Laisser agiter une nuit à température ambiante. Amener à pH 8 avec NaOH 1N. Extraire avec CH₂Cl₂. Sécher avec Mg SO₄. Evaporer le solvant. Récupérer le produit. Extraire avec l'éthyl méthyl cétone. Sécher sur Mg SO₄. Evaporer le solvant. Purifier le produit sur 30 g de silice. Eluer avec CH₂Cl₂/Me OH 95 : 5. On obtient le produit de l'exemple 78.
- - Masse moléculaire: : 245,28
- - Point de fusion: : 171-175°C
- - solvants de recristallisation: : CH₃CN, MeOH

### EXEMPLES 79 A 87

En procédant comme dans les exemples 76 à 78 mais en utilisant le composé naphtylalkylamide convenablement substitué, on obtient les composés des exemples suivants :
- **EXEMPLES 79 :**: **N-[2-(7-METHOXY 3-ACETOXY NAPHT-1-YL)ETHYL]PROPIONAMIDE**
- **EXEMPLES 80 :**: **N-[2-(7-METHOXY 3-HYDROXY NAPHT-1-YL)ETHYL]PROPIONAMIDE**
- **EXEMPLES 81 :**: **N-[2-(3,7-DIHYDROXY NAPHT-1-YL)ETHYL]PROPIONAMIDE**
- **EXEMPLES 82 :**: **N-[2-(7-METHOXY 3-ACETOXY NAPHT-1-YL)ETHYL]CYCLOPROPYLCARBOXAMIDE**
- **EXEMPLES 83 :**: **N-[2-(7-METHOXY 3-HYDROXY NAPHT-1-YL)ETHYL]CYCLOPROPYLCARBOXAMIDE**
- **EXEMPLE 84 :**: **N-[2-(3,7-DIHYDROXY NAPHT-1-YL)ETHYL]CYCLOPROPYLCARBOXAMIDE**
- **EXEMPLE 85 :**: **N-[2-(7-METHOXY 3-ACETOXY NAPHT-1-YL)ETHYL]CYCLOBUTYLCARBOXAMIDE**
- **EXEMPLE 86 :**: **N-[2-(7-METHOXY 3-HYDROXY NAPHT-1-YL)ETHYL]CYCLOBUTYLCARBOXAMIDE**
- **EXEMPLE 87 :**: **N-[2-(3,7-DIHYDROXY NAPHT-1-YL)ETHYL]CYCLOBUTYL CARBOXAMIDE**
- **EXEMPLE 88 :**: **N-[2-(7-METHOXY 3-BENZYL NAPHT-1-YL)ETHYL]ACETAMIDE**

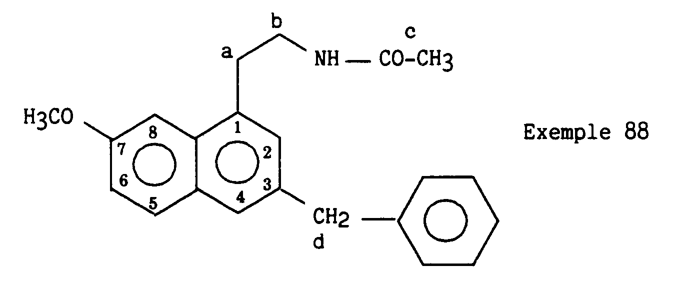

Une solution de 72 mg de chlorure mercurique dans 5,5 cm³ d'eau est ajoutée à 3,6 g de zinc en poudre. Agiter pendant 30 minutes. Laisser décanter et éliminer l'eau. Ajouter alors 3,6 cm³ d'eau à cet amalgame, puis 3,6 cm³ d'acide chlorhydrique concentré, puis 5,26 mmoles de N-[2-(7-méthoxy 3-benzoyl napht-1-yl)éthyl]acétamide préparé selon l'exemple 1, et 25 cm³ de toluène. Porter à reflux pendant 2 heures, extraire la phase organique, laver à l'eau, sécher sur sulfate de magnésium, filtrer et évaporer à sec.

On obtient le composé de l'exemple 88.
- Rendement : 35 %
- Point de fusion : 85°-87°C
- Solvant de recristallisation : Toluène
- Masse moléculaire : 333,411 pour C₂₂ H₂₃ NO₂
- Microanalyse :

| | **C %** | **H %** | **N %** |
|---|---|---|---|
| Calculé | 79,24 | 6,95 | 4,20 |
| Trouvé | 78,93 | 6,94 | 4,22 |

- Infra-Rouge :
ν N-H (amide) : 3220 cm⁻¹
ν C = O (amide) : 1610 cm⁻¹
ν C = O (aromatique) : 1590 cm⁻¹

- RMN (DMSO,d₆)
1,80 ppm (singulet, 3 H, (H_{c}))
3,00 - 3,45 ppm (massif, 4 H, (Hₐ, H_{b}))
3,90 ppm (singulet, 3 H, (O CH₃))
4,00 ppm (singulet, 2 H, (H_{d}))
7,00 - 7,80 ppm (massif, 10 H, H aromatique)
8,00 ppm (signal, 1H, (NH))

### EXEMPLES 89 A 114 :

En procédant comme dans l'exemple 88 mais en partant des exemples 2 à 26, on obtient les composés des exemples suivants :
- **EXEMPLE 89 :**: **N-[2-(7-METHOXY 3-BENZYL NAPHT-1-YL)ETHYL]PROPIONAMIDE**
- **EXEMPLE 90 :**: **N-[2-(7-METHOXY 3-BENZYL NAPHT-1-YL)ETHYL]BUTYRAMIDE**
- **EXEMPLE 91 :**: **N-[2-(7-METHOXY 3-BENZYL NAPHT-1-YL)ETHYL]PENTANAMIDE**
- **EXEMPLE 92 :**: **N-[2-(7-METHOXY 3-BENZYL NAPHT-1-YL)ETHYL]HEXANAMIDE**
- **EXEMPLE 93 :**: **N-[2-(7-METHOXY 3-BENZYL NAPHT-1-YL)ETHYL]CYCLOPROPYLCARBOXAMIDE**
- **EXEMPLE 94 :**: **N-[2-(7-METHOXY 3-BENZYL NAPHT-1-YL)ETHYL**]**CYCLOBUTYLCARBOXAMIDE**
- **EXEMPLE 95 :**: **N-[2-(7-METHOXY 3-BENZYL NAPHT-1-YL)ETHYL**]**CYCLOPENTYLCARBOXAMIDE**
- **EXEMPLE 96 :**: **N-[2-(7-METHOXY 3-BENZYL NAPHT-1-YL)ETHYL**]**CYCLOHEXYLCARBOXAMIDE**
- **EXEMPLE 97 :**: **N-[2-(7-METHOXY 3-BENZYL NAPHT-1-YL)ETHYL] N'-METHYLUREE**
- **EXEMPLE 98 :**: **N-[2-(7-METHOXY 3-BENZYL NAPHT-1-YL)ETHYL] N'-ETHYLUREE**
- **EXEMPLE 99 :**: **N-[2-(7-METHOXY 3-BENZYL NAPHT-1-YL)ETHYL] N'-PROPYLUREE**
- **EXEMPLE 100 :**: **N-[2-(7-METHOXY 3-BENZYL NAPHT-1-YL)ETHYL] N'-HEXYLUREE**
- **EXEMPLE 101 :**: **N-[2-(7-METHOXY 3-BENZYL NAPHT-1-YL)ETHYL] N'-CYCLOPROPYLUREE**
- **EXEMPLE 102 :**: **N-[2-(7-METHOXY 3-BENZYL NAPHT-1-YL)ETHYL] N'-CYCLOHEXYLUREE**
- **EXEMPLE 103 :**: **N-[2-(3-BENZYL NAPHT-1-YL)ETHYL]ACETAMIDE**
- **EXEMPLE 104 :**: **N-[2-(7-ETHOXY 3-BENZYL NAPHT-1-YL)ETHYL]ACETAMIDE**
- **EXEMPLE 105 :**: **N-[2-(7-PROPOXY 3-BENZYL NAPHT-1-YL)ETHYL]ACETAMIDE**
- **EXEMPLE 106 :**: **N-[2-(7-PENTOXY 3-BENZYL NAPHT-1-YL)ETHYL]ACETAMIDE**
- **EXEMPLE 107 :**: **N-[2-(7-PROPARGYLOXY 3-BENZYL NAPHT-1-YL)ETHYL]ACETAMIDE**
- **EXEMPLE 108 :**: **N-[2-(7-PROPARGYLOXY 3-BENZYL NAPHT-1-YL)ETHYL]ACETAMIDE**
- **EXEMPLE 109 :**: **N-[2-(7-CYCLOPROPYLMETHYLOXY 3-BENZYL NAPHT-1-YL)ETHYL]ACETAMIDE**
- **EXEMPLE 110 :**: **N-[2-(7-CYCLOHEXYLOXY 3-BENZYL NAPHT-1-YL)ETHYL]ACETAMIDE**
- **EXEMPLE 111 :**: **N-[2-(7-CYCLOHEXEN-2-YLOXY 3-BENZYL NAPHT-1-YL)ETHYL]ACETAMIDE**
- **EXEMPLE 112 :**: **N-[2-(7-BENZYLOXY-3-BENZYL NAPHT-1-YL)ETHYL]ACETAMIDE**
- **EXEMPLE 113 :**: **N-[2-(3-BENZYL NAPHT-1-YL)ETHYL]CYCLOPROPYLCARBOXAMIDE**
- **EXEMPLE 114 :**: **N-[2-(3-BENZYL NAPHT-1-YL)ETHYL]CYCLOBUTYLCARBOXAMIDE**
- **EXEMPLE 115 :**: **N-[2-(7-METHOXY 3-ETHYL NAPHT-1-YL)ETHYL]ACETAMIDE**

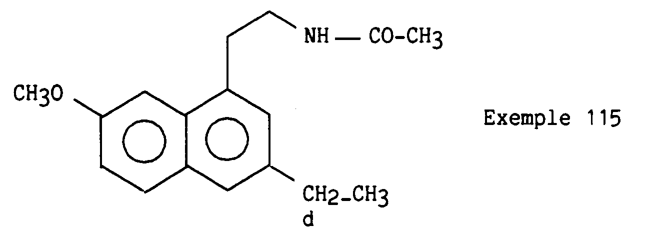

A partir du N-[2-(7-méthoxy 3-acétyl napht-l-yl)éthyl]acétamide préparé selon l'exemple 49, et en procédant de la même façon que dans l'exemple 88, on obtient le composé de l'exemple 115. Masse moléculaire : 271,363 pour C₁₇ H₂₁ NO₂ Point de fusion : 104 - 105 °C
- Microanalyse :

| | **C %** | **H %** | **N %** |
|---|---|---|---|
| Calculé | 75,25 | 7,80 | 5,16 |
| Trouvé | 74,99 | 8,12 | 5,08 |

### EXEMPLES 116 A 141 :

En procédant comme dans l'exemple 115 mais en partant des exemples 50 à 75, on obtient les composés des exemples suivants :
- **EXEMPLE 116 :**: **N-[2-(7-METHOXY 3-ETHYL NAPHT-1-YL)ETHYL]PROPIONAMIDE**
- **EXEMPLE 117 :**: **N-[2-(7-METHOXY 3-ETHYL NAPHT-1-YL)ETHYL]BUTYRAMIDE**
- **EXEMPLE 118 :**: **N-[2-(7-METHOXY 3-ETHYL NAPHT-1-YL)ETHYL]PENTANAMIDE**
- **EXEMPLE 119 :**: **N-[2-(7-METHOXY 3-ETHYL NAPHT-1-YL)ETHYL]HEXANAMIDE**
- **EXEMPLE 120 :**: **N-[2-(7-METHOXY 3-ETHYL NAPHT-1-** **YL)ETHYL**]**CYCLOPROPYLCARBOXAMIDE**

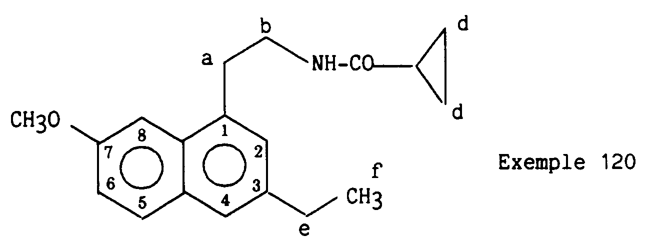

- - Rendement: : 38 %
- - Point de fusion: : 116°-118°C
- - Solvant de recristallisation: : Cyclohexane
- - Masse moléculaire: : 297,381 pour C₁₉ H₂₃ NO₂
- - Infra-Rouge: :
ν N-H (amide) : 3240 cm⁻¹
ν C = O (amide) : 1620 cm⁻¹
- - RMN (DMSO,d₆): 0,70 ppm (multiplet, 4 H, (H_{d}))
1,25 ppm (triplet, 3 H, (H_{f}))
1,60 ppm (multiplet, 1 H, (H_{c}))
2,70 ppm (quadruplet, 2 H, (Hₑ))
3,10 ppm (triplet, 2 H, (Hₐ)
3,35 ppm (multiplet, 2 H, (H_{b}))
3,90 ppm (singulet, 3 H, (OCH₃))
7,10 ppm (doublet, 1 H, (H₆), J₆₋₅ = 8,33 Hz)
7,15 ppm (singulet, 1 H, (H₈))
7,50 ppm (singulet, 2 H, (H₂, H₄))
7,70 ppm (doublet, 1 H, (H₅), J₅₋₆ = 8,33 Hz))
8,20 ppm (triplet, 1 H, (NH))

- **EXEMPLE 121 :**: **N-[2-(7-METHOXY 3-ETHYL NAPHT-1-YL)ETHYL]CYCLOBUTYLCARBOXAMIDE**
- **EXEMPLE 122 :**: **N-[2-(7-METHOXY 3-ETHYL NAPHT-1-YL)ETHYL**]**CYCLOPENTYLCARBOXAMIDE**
- **EXEMPLE 123 :**: **N-[2-(7-METHOXY 3-ETHYL NAPHT-1-YL)ETHYL**]**CYCLOHEXYLCARBOXAMIDE**
- **EXEMPLE 124 :**: **N-[2-(7-METHOXY 3-ETHYL NAPHT-1-YL)ETHYL] N'-METHYLUREE**
- **EXEMPLE 125 :**: **N-[2-(7-METHOXY 3-ETHYL NAPHT-1-YL)ETHYL] N'-ETHYLUREE**
- **EXEMPLE 126 :**: **N-[2-(7-METHOXY 3-ETHYL NAPHT-1-YL)ETHYL] N'-PROPYLUREE**
- **EXEMPLE 127 :**: **N-[2-(7-METHOXY 3-ETHYL NAPHT-1-YL)ETHYL] N'-HEXYLUREE**
- **EXEMPLE 128 :**: **N-[2-(7-METHOXY 3-ETHYL NAPHT-1-YL)ETHYL] N'-CYCLOPROPYLUREE**
- **EXEMPLE 129 :**: **N-[2-(7-METHOXY 3-ETHYL NAPHT-1-YL)ETHYL] N'-CYCLOHEXYLUREE**
- **EXEMPLE 130 :**: **N-[2-(3-BENZOYL NAPHT-1-YL)ETHYL]ACETAMIDE**
- **EXEMPLE 131 :**: **N-[2-(7-ETHOXY 3-ETHYL NAPHT-1-YL)ETHYL]ACETAMIDE**
- **EXEMPLE 132 :**: **N-[2-(7-PROPOXY 3-ETHYL NAPHT-1-YL)ETHYL]ACETAMIDE**
- **EXEMPLE 133 :**: **N-[2-(7-PENTOXY 3-ETHYL NAPHT-1-YL)ETHYL]ACETAMIDE**
- **EXEMPLE 134 :**: **N-[2-(7-ALLYLOXY 3-ETHYL NAPHT-1-YL)ETHYL]ACETAMIDE**
- **EXEMPLE 135 :**: **N-[2-(7-PROPARGYLOXY 3-ETHYL NAPHT-1-YL)ETHYL]ACETAMIDE**
- **EXEMPLE 136 :**: **N-[2-(7-CYCLOPROPYLMETHYLOXY 3-ETHYL NAPHT-1-YL)ETHYL]ACETAMIDE**
- **EXEMPLE 137 :**: **N-[2-(7-CYCLOHEXYLOXY 3-ETHYL NAPHT-1-YL)ETHYL]ACETAMIDE**
- **EXEMPLE 138 :**: **N-[2-(7-CYCLOHEXEN-2-YLOXY 3-ETHYL NAPHT-1-YL)ETHYL]ACETAMIDE**
- **EXEMPLE 139 :**: **N-[2-(7-BENZYLOXY-3-ETHYL NAPHT-1-YL)ETHYL]ACETAMIDE**
- **EXEMPLE 140 :**: **N-[2-(3-ETHYL NAPHT-1-YL)ETHYL]CYCLOPROPYLCARBOXAMIDE**
- **EXEMPLE 141 :**: **N-[2-(3-ETHYL NAPHT-1-YL)ETHYL]CYCLOBUTYLCARBOXAMIDE**
- **EXEMPLE 142 :**: **N-[2-(7-METHOXY 3-CYCLOPROPYLMETHYL NAPHT-1-YL)ETHYL]ACETAMIDE**

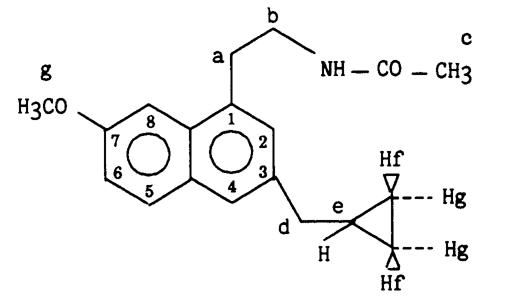

A partir du N-[2-(7-méthoxy 3-cyclopropylcarbonyl napht-1-yl)éthyl]acétamide préparé selon l'exemple 27, et en procédant de la même façon que dans l'exemple 88, on obtient le composé de l'exemple 142.
- Rendement : 37,5 %
- Point de fusion : 89°-90°C
- Solvant de recristallisation : Cyclohexane
- Masse moléculaire : 301,885 pour C₁₉ H₂₃ NO₂ + 1/4 H₂O
- Microanalyse :

| | **C %** | **H %** | **N %** |
|---|---|---|---|
| Calculé | 75,58 | 7,84 | 4,63 |
| Trouvé | 75,65 | 7,74 | 4,49 |

- Infra-Rouge :
ν N-H (amide) : 3240 cm⁻¹
ν C = O (amide) : 1625 cm⁻¹

- RMN (DMSO,d₆)
0,20 ppm (multiplet, 2 H, (H_{g}))
0,50 ppm (multiplet, 2 H, (H_{f}))
1,05 ppm (multiplet, 1 H, (Hₑ))
1,85 ppm (singulet, 3 H, (H_{c}))
2,60 ppm (doublet, 2 H, (H_{d}), J = 6,84 Hz)
3,10 ppm (triplet, 2 H, (Hₐ))
3,30 ppm (multiplet, 2 H, (H_{b}))
3,90 ppm (singulet, 1 H, (OCH₃))
7,15 ppm (doublet dédoublé, 1 H, (H₆), J₆₋₅ = 8,90 Hz J₆₋₈ = 2,40 Hz)
7,25 ppm (singulet, 1 H, (H₈), J₈₋₆ = 2,40 Hz)
7,55 ppm (multiplet, 2 H, (H₂, H₄ Hz))
7,80 ppm (doublet, 1 H, (H₅), J₅₋₆ = 8,90 Hz)
8,10 ppm (triplet, 1 H, (NH))

### EXEMPLE 143 : N-[2-(7-METHOXY 3-PROPYL NAPHT-1-YL)ETHYL]ACETAMIDE

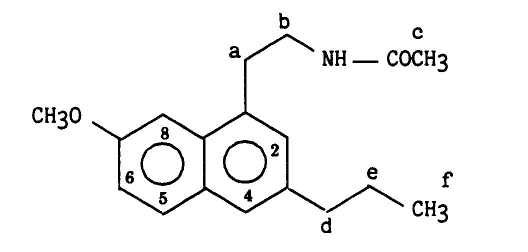

A partir du N-[2-(7-méthoxy 3-propionyl napht-1-yl)éthyl]acétamide préparé selon l'exemple 38, et en procédant de la même façon que dans l'exemple 88, on obtient le composé de l'exemple 143.
- Rendement : 60 %
- Point de fusion : 80°-82°C
- Solvant de recristallisation : Ether de pétrole
- Masse moléculaire : 285,371 pour C₁₈ H₂₃ NO₂
- Microanalyse :

| | **C %** | **H %** | **N %** |
|---|---|---|---|
| Calculé | 75,75 | 8,12 | 4,91 |
| Trouvé | 75,46 | 7,95 | 4,90 |

- Infra-Rouge :
ν N-H (amide) : 3230 cm⁻¹
ν C = O (amide) : 1620 cm⁻¹
ν C = C (aromatique) : 1600 cm⁻¹

- RMN (DMSO,d6)
0,90 ppm (triplet, 3 H, (H_{f}), J = 8,35 Hz)
1,65 ppm (multiplet, 2 H, (Hₑ))
1,80 ppm (singulet, 3 H, (H_{c}))
2,65 ppm (triplet, 2 H, (H_{d}), J = 7,49 Hz)
3,10 ppm (triplet, 2 H, (Hₐ))
3,30 ppm (multiplet, 2 H, (H₂))
3,90 ppm (singulet, 3 H, (OCH₃))
7,10 ppm (multiplet, 2 H, (H₆, H₂))
7,50 ppm (singulet, 1 H, (H₄))
7,55 ppm (doublet, 1 H, (H₈), J₈₋₆ = 1,95 Hz)
7,75 ppm (doublet, 1 H, (H₅), J₅₋₆ = 8,39 Hz)
8,10 ppm (triplet, 1 H, (NH))

### EXEMPLE 144 A 148 :

En procédant comme dans les exemples 1, 49, 50, 115 et 120 mais en partant des composés thiocarboxamides correspondants, on obtient les composés des exemples suivants :
- **EXEMPLE 144 :**: **N-[2-(7-METHOXY 3-BENZOYL NAPHT-1-YL)ETHYL]THIOACETAMIDE**
- **EXEMPLE 145 :**: **N-[2-(7-METHOXY 3-BENZOYL NAPHT-1-YL)ETHYL]THIOACETAMIDE**
- **EXEMPLE 146 :**: **N-[2-(7-METHOXY 3-ACETYL NAPHT-1-YL)ETHYL]CYCLOPROPYLTHIOCARBOXAMIDE**
- **EXEMPLE 147 :**: **N-[2-(7-METHOXY 3-ETHYL NAPHT-1-YL)ETHYL]THIOACETAMIDE**
- **EXEMPLE 148 :**: **N-[2-(7-METHOXY 3-ETHYL NAPHT-1-YL)ETHYL]CYCLOPROPYLTHIOCARBOXAMIDE**

### ETUDE PHARMACOLOGIQUE DES DERIVES DE L'INVENTION

### EXEMPLE A : ETUDE DE LA TOXICITE AIGUE

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL50 entraînant la mort de 50% des animaux, a été évaluée. La DL50 des produits testés est supérieure à 1000 mg.kg⁻¹ pour les composés étudiés ce qui indique la faible toxicité des composés de l'invention.

### EXEMPLE B : TEST DES QUATRE PLAQUES

Les produits de l'invention sont administrés par voie oesophagienne à des lots de dix souris. Un lot reçoit du sirop de gomme.

30 minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute. Après administration, les composés de l'invention augmentent de façon significative le nombre de passages ce qui montre l'activité anxiolytique des dérivés de l'invention.

### EXEMPLE C : ACTIVITE DES PRODUITS DE L'INVENTION SUR LA MICROCIRCULATION ISCHEMIQUE

L'étude expérimentale a été réalisée sur les muscles crémasters de rats mâles (Sprague-Dawley) après ligature de l'artère iliaque commune.

Les muscles ont été placés dans une chambre transparente, perfusés par une solution de tampon bicarbonate équilibrée par un mélange gazeux CO2/N2 5/95%. La vélocité des globules rouges et le diamètre des artérioles de premier ou second ordre irriguant le crémaster ont été mesurés, le flux sanguin artériolaire a été calculé. Des informations identiques ont été obtenues pour quatre types de veinules.

On a effectué le même type de mesure simultanément :
- sur le crémaster perfusé normalement,
- sur le crémaster sous ligature, c'est-à-dire le crémaster ischémié 2, 7, 14 et 21 jours après ligature.

Deux groupes d'animaux ont été étudiés :
- un groupe témoin sans traitement,
- un groupe traité per os par un produit de l'invention, à raison de 0,1 mg.kg-1 par jour.

On n'a constaté aucune différence dans la vélocité des globules ni dans le diamètre des vaisseaux dans les muscles crémasters normalement irrigués chez les animaux traités par rapport aux témoins.

Par contre, au niveau du muscle crémaster ischémié, le diamètre moyen des artérioles était amélioré chez les animaux traités par rapport aux témoins. La vélocité des globules rouges était normalisée par un traitement de 21 jours.

En fait, chez les animaux traités, la vélocité des globules rouges et le débit sanguin mesurés 7 jours après la ligature, ne présentent pas de différence significative avec les valeurs obtenues dans le crémaster non ischémié. Ces résultats sont obtenus sans modification de la pression artérielle.

Ces résultats indiquent que le traitement chronique par un composé de l'invention améliore la microcirculation et l'irrigation sanguine des territoires ischémiés.

### EXEMPLE D : STIMULATION DES REPONSES IMMUNITAIRES

A des groupes de six souris, on a administré des globules rouges de moutons. Ces groupes de souris ont ensuite été traités par voie sous cutanée par les composés de l'invention pendant six jours et un groupe témoin a été traité par un placébo. Les souris sont ensuite laissées au repos pendant quatre semaines puis ont ensuite reçu une injection de rappel de globules rouges de mouton sans recevoir de nouvelles administrations de produit de l'invention. La réponse immunitaire a été évaluée 3 jours après l'injection de rappel. Elle est statistiquement accrue dans le groupe traité par les composés de l'invention.

### EXEMPLE E : EFFETS DES COMPOSES DE L'INVENTION SUR LES RYTHMES CIRCADIENS D'ACTIVITE LOCOMOTRICE DU RAT

L'implication de la mélatonine dans l'entraînement, par l'alternance jour/nuit, de la plupart des rythmes circadiens physiologiques, biochimiques et comportementaux a permis d'élaborer un modèle pharmacologique utile pour rechercher des ligands mélatoninergiques.

Les effets des composés de l'invention sont testés sur de nombreux paramètres et en particulier sur les rythmes circadiens d'activité locomotrice qui représentent un marqueur fiable de l'activité de l'horloge circadienne endogène.

Dans cette étude, on évalue les effets des composés testés sur un modèle expérimental particulier, à savoir le rat placé en isolement temporel (obscurité permanente).

### PROTOCOLE :

Des rats mâles Long Evans agés de un mois sont soumis dès leur arrivée au laboratoire à un cycle lumineux de 12 h de lumière par 24 h (LD 12:12). Après 2 à 3 semaine d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12:12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux
- disparition de l'entraînement des rythmes en obscurité permanente
- entraînement par l'administration quotidienne de la molécule ; effet transitoire ou durable.

Un logiciel permet de mesurer la durée et l'intensité de l'activité, la période du rythme chez les animaux en libre cours et pendant le traitement.

### RESULTATS :

Les résultats des tests montrent clairement que les composés de l'invention sont d'interessants agents thérapeutiques pour le traitement des désordres du rythme circadien.

### EXEMPLE F : MISE EN EVIDENCE DE L'ACTIVITE ANALGESIQUE

L'activité sur la douleur a été recherchée chez la souris (23-25 g) selon un protocole dérivé de la technique décrite par SIEGMUND (SIEGMUND E.A., R.A. CADMUS & GOLU, J. Pharm. Exp. Ther. 119, 1874, 1954). Les souris, réparties par randomisation en lots de 12 animaux, ont reçu le traitement par voie orale (excipient pour les témoins) 1 heure avant l'injection intra-péritonéale d'une solution hydroalcoolique de phényl-p-benzoquinone (Sigma) à 0,02%. Les étirements sont dénombrés entre la 5ème et 10ème minute après l'injection.

Il est apparu que les composés de l'invention possèdent une activité analgésique.

### EXEMPLE G : TEST DE BINDING AUX RECEPTEURS DE LA MELATONINE SUR LA PARS TUBERALIS DE MOUTON

Le binding aux récepteurs de la mélatonine des composés de l'invention a été réalisé selon les techniques classiques sur la Pars Tuberalis de mouton, tel que décrit dans "Journal of Neuroendocrinology, vol. 1, N°1, 1989". Il apparaît que les composés de l'invention se lient de façon très spécifique aux récepteurs de la mélatonine, avec une affinité supérieure à celle de la mélatonine elle-même.

### EXEMPLE H : TEST DE COMPETITION AUX RECEPTEURS DE LA MELATONINE SUR DES MEMBRANES DE CELLULES DU CERVEAU DE POULET GALLUS DOMESTICUS

Les animaux utilisés sont des poulets (Gallus domesticus) agés de 12 jours. Ils sont sacrifiés entre 13 et 17 heures le jour de leur arrivée. Les cerveaux sont rapidement prélevés et congelés à - 200°C puis conservés à -80°C. Les membranes sont préparées selon la méthode décrite par Yuan et Pang (1991). La fixation de la mélatonine sur les membranes est réalisée selon un protocole établi d'après Rivkees et al. (1989). Brièvement, la [¹²⁵I] mélatonine est incubée en présence des membranes dans une solution tamponnée à pH7.4 pendant 60 min à 25°C. A l'issue de cette période, la suspension membranaire est filtrée (Whatman GF/C). La radioactivité retenue sur le filtre est déterminée à l'aide d'un compteur à scintillation liquide Beckman® LS 6000.

Les produits utilisés sont :
. 2[¹²⁵I] mélatonine
. mélatonine
. produits courants
. molécules originales

En screening primaire, les molécules sont testées à 2 concentrations (10⁻⁷ et 10⁻⁵M). Chaque résultat est la moyenne de n=3 mesures indépendantes. Les molécules actives retenues d'après les résultats du screening primaire ont fait l'objet d'une détermination quantitative de leur efficacité (IC₅₀). Elles sont utilisées à 10 concentrations différentes.

Ainsi les valeurs d'IC₅₀ trouvées pour les composés préférés de l'invention, qui correpondent aux valeurs de l'affinité montrent que la liaison des composés testés aux récepteurs mélatoninergiques est, de façon surprenante, très puissante.

### EXEMPLE I : COMPOSITION PHARMACEUTIQUE : COMPRIMES

Comprimés dosés à 5 mg de N-[2-(7-méthoxy 3-éthyl napht-1-yl)éthyl]acétamide . Formulation pour préparer 1000 comprimés.

| | |
|---|---|
| N-[2-(7-méthoxy 3-éthyl napht-1-yl)éthyl]acétamide | 5 g |
| Amidon de blé | 15 g |
| Amidon de maïs | 15 g |
| Lactose | 15 g |
| Stéarate de magnésium | 2 g |
| Silice | 1 g |
| Hydroxypropyl cellulose | 2 g |

## Revendications

1. Composés de formule générale (I) : 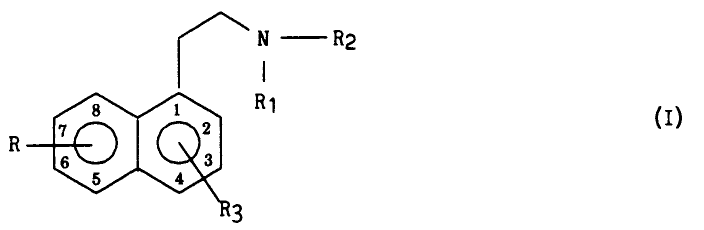 dans laquelle :
• R représente un hydrogène ou un radical choisi parmi alkyle, alkyle substitué et -O-R' ; avec R' représentant un hydrogène ou un radical alkyle, alkyle substitué, cycloalkyle, cycloalkyle substitué, cycloalkylalkyle, cycloalkylalkyle substitué, phényle, phényle substitué, phénylalkyl, phénylalkyle substitué, diphénylalkyle, ou diphénylalkyle substitué ;
• R₁ représente un hydrogène ou un alkyle ;
• R₂ signifie
a) 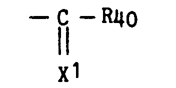 dans lequel X¹ représente un atome de soufre ou d'oxygène et R₄₀ représente un hydrogène ou un radical choisi parmi alkyle, alkyle substitué, cycloalkyle, cycloalkyle substitué, cycloalkylalkyle, cycloalkylalkyle substitué, alkényle, alkényle substitué, alkynyle et alkynyle substitué ;
ou
b) 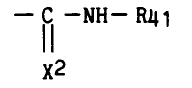 dans laquelle X² représente un soufre ou un oxygène et R₄₁ représente un hydrogène ou un radical choisi parmi alkyle, cycloalkyle et cycloalkylalkyle ;
• R₃ représente un radical choisi parmi :
- (C₂-C₆) alkyle,
- (C₂-C₆) alkyle substitué,
- cycloalkylalkyle,
- cycloalkylalkyle substitué,
- alkényle,
- alkényle substitué,
- alkynyle,
- alkynyle substitué,
- hydroxyle en position 3,
- R₅-alkyle-, avec R₅ étant substitué ou non substitué et représentant un radical choisi parmi pyridyle, phényle, naphtyle, thiényle, furyle, pyrimidyle, indolyle, benzofuryle, benzothiényle, quinolyle et isoquinolyle ;
- R₆-CO-O- ,
- et R₆-CO- ,
avec R₆ représentant un radical choisi parmi (C₁-C₅)alkyle, (C₁-C₅)alkyle substitué, cycloalkyle, cycloalkyle substitué, cycloalkyl(C₁-C₅)alkyle, cycloalkyl(C₁-C₅)alkyle substitué, R₅- et R₅-alkyle- où R₅ étant non substitué ou substitué est tel que défini précédemment,
étant entendu que le composé de formule générale (I) ne peut pas être le N-{2-[(4-acétyl)napht-1-yl]éthyl}acétamide,
- les termes "alkyle" et "alkoxy" désignant, sauf mention contraire, des groupements linéaires ou ramifiés de 1 à 6 atomes de carbone,
- les termes "alkényle" et "alkynyle" désignant des groupements insaturés, linéaires ou ramifiés, de 2 à 6 atomes de carbone,
- le terme "cycloalkyle" désignant un groupement cyclique de 3 à 8 atomes de carbone,
- le terme "substitué" affecté à "alkyle", "alkoxy", "alkényle", "alkynyle", "cycloalkyle" ou "cycloalkylalkyle" signifie que ces groupements sont substitués par un ou plusieurs radicaux choisi parmi alkyle, alkoxy et halogène ;
- le terme "substitué" associé à R₅ et à "phényle", "phénylalkyle" ou "diphénylalkyle" signifie que ces groupements sont substitués par un ou plusieurs radicaux choisis parmi alkyle, alkoxy, halogène, hydroxy et trifluorométhyle ;
- leurs énantiomères et diastéreoisomères,
- et leurs sels d'addition à un acide ou à une base pharmaceutiquement acceptable.

2. Composé selon la revendication 1 qui est le N-[2-(7-méthoxy 3-benzoyl napht-1-yl)éthyl]acétamide

3. Composé selon la revendication 1 qui est le N-[2-(7-méthoxy 3-acétyl napht-1-yl)éthyl]acétamide.

4. Composé selon la revendication 1 qui est le N-[2-(7-méthoxy 3-acétyl napht-1-yl)éthyl]cyclopropylcarboxamide.

5. Composé selon la revendication 1 qui est le N-[2-(7-méthoxy 3-éthyl napht-1-yl)éthyl]acétamide.

6. Composé selon la revendication 1 qui est le N-[2-(7-méthoxy 3-propyl napht-1-yl)éthyl]acétamide.

7. Procédé de préparation des composés de formule (I) selon la revendication 1, dans lequel on fait réagir un composé de formule (II) : 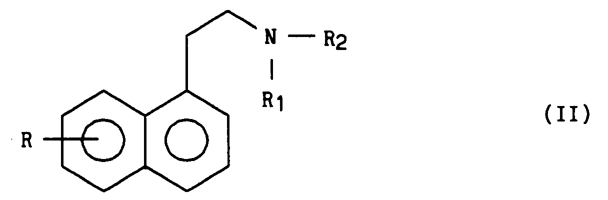 dans laquelle R, R₁, et R₂ sont tels que définis dans la revendication 1, avec un composé de formule (III) :
R₆-COCl (III)
dans laquelle R₆ est tel que défini dans la revendication 1, pour obtenir un composé de formule (Ia) correspondant : 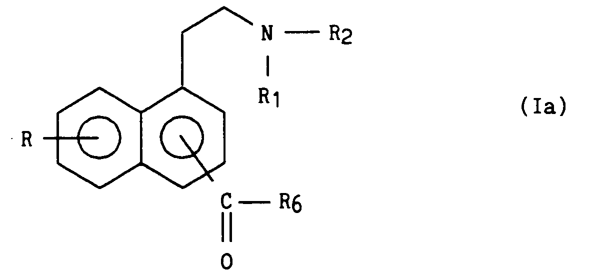 dans laquelle R, R₁, R₂ et R₆ sont tels que définis précédemment, qui peut être, si on le désire,
- soit soumis à une oxydation par une réaction de Baeyer-Villiger, pour obtenir un composé de formule (Ib) correspondant : 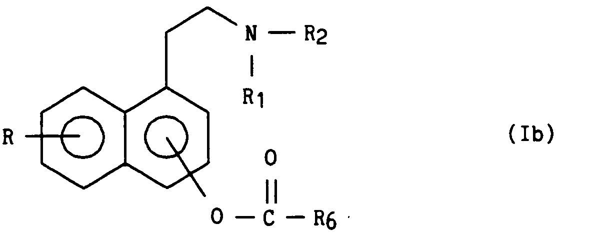 dans laquelle R, R₁, R₂ et R₆ sont tels que définis précédemment, puis si le substituant -O-CO-R₆ est en position 3 soumis à une saponification en présence d'hydroxyde de sodium, pour obtenir un composé de formule (Ic) correspondant : 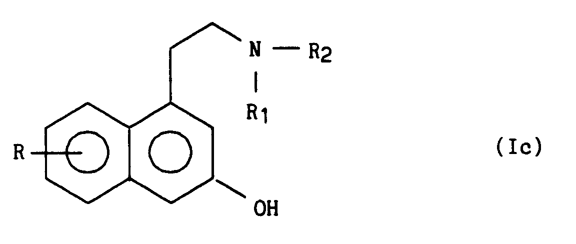 dans laquelle R, R₁, R₂ sont tels que définis précédemment,
- soit soumis à une réduction par le mercure et le zinc, en présence de toluène et d'acide chlorhydrique, pour obtenir un composé de formule (Id) correspondant : 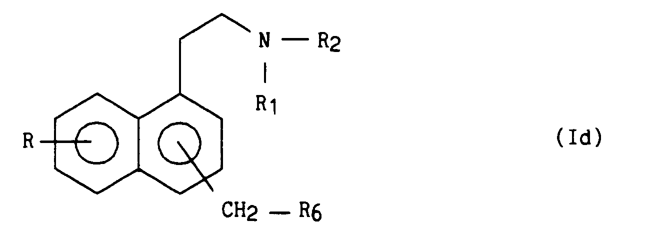 dans laquelle R, R₁, R₂ et R₆ sont tels que définis précédemment,
les composés de formule (Ia), (Ib), (Ic), et (Id) formant l'ensemble des composés de formule (I),
composés de formule (I) qui peuvent être,
- purifiés suivant une ou plusieurs méthodes de purification choisies parmi la cristallisation, la chromatographie sur gel de silice, l'extraction, la filtration, et le passage sur charbon ou résine,
- séparés, le cas échéant, sous forme pure ou sous forme de mélange, en leurs éventuels énantiomères ou diastéréoisomères,
- ou salifiés par un acide ou une base pharmaceutiquement acceptable.

8. Compositions pharmaceutiques contenant les produits de formule (I) selon la revendication 1 ou le cas échéant un de leurs sels d'addition à un acide ou une base pharmaceutiquement acceptable en combinaison avec un ou plusieurs excipients pharmaceutiquement acceptables.

9. Compositions pharmaceutiques selon la revendication 8 sont utiles pour le traitement des troubles mélatoninergiques.

10. Compositions pharmaceutiques selon la revendication 9 utiles dans les traitements des dépressions saisonnières, des troubles du sommeil, des pathologies cardiovasculaires, des insomnies et fatigues dues aux décalages horaires, et des troubles de l'appétit et de l'obésité.

## Claims

1. Compounds of the general formula (I): 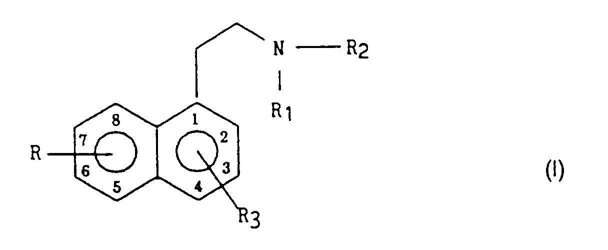 wherein :
• R represents a hydrogen atom or a radical selected from alkyl, substituted alkyl and -O-R', R' representing a hydrogen atom or an alkyl, substituted alkyl, cycloalkyl, substituted cycloalkyl, cycloalkylalkyl, substituted cycloalkylalkyl, phenyl, substituted phenyl, phenylalkyl, substituted phenylalkyl, diphenylalkyl, or substituted diphenylalkyl radical;
• R₁ represents a hydrogen atom or an alkyl radical;
• R₂ represents
a) 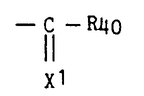 wherein X¹ represents a sulphur or oxygen atom and R₄₀ represents a hydrogen atom or a radical selected from alkyl, substituted alkyl, cycloalkyl, substituted cycloalkyl, cycloalkylalkyl, substituted cycloalkylalkyl, alkenyl, substituted alkenyl, alkynyl and substituted alkynyl;
or b) 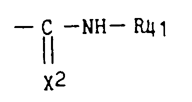 wherein X² represents a sulphur or oxygen atom and R₄₁ represents a hydrogen atom or a radical selected from alkyl, cycloalkyl and cycloalkylalkyl;
• R₃ represents a radical selected from :
- (C₂-C₆)alkyl,
- substituted (C₂-C₆)alkyl,
- cycloalkylalkyl,
- substituted cycloalkylalkyl,
- alkenyl,
- substituted alkenyl,
- alkynyl,
- substituted alkynyl,
- hydroxy in the 3 position,
- R₅-alkyl-, R₅ being substituted or unsubstituted and representing a radical selected from pyridyl, phenyl, naphthyl, thienyl, furyl, pyrimidyl, indolyl, benzofuryl, benzothienyl, quinolyl and isoquinolyl;
- R₆-CO-O-, and
- R₆-CO-,
R₆ representing a radical selected from (C₁-C₅)alkyl, substituted (C₁-C₅)alkyl, cycloalkyl, substituted cycloalkyl, cycloalkyl(C₁-C₅)alkyl, substituted cycloalkyl(C₁-C₅)alkyl, R₅- and R₅-alkyl- in which R₅ is unsubstituted or substituted and is as defined above,
with the proviso that the compound of the general formula (I) cannot be N-{2-[4-(acetyl)naphth-1-yl]ethyl}acetamide,
- the terms "alkyl" and "alkoxy" denoting, unless indicated otherwise, linear or branched groups having from 1 to 6 carbon atoms,
- the terms "alkenyl" and "alkynyl" denoting unsaturated linear or branched groups having from 2 to 6 carbon atoms,
- the term "cycloalkyl" denoting a cyclic group having from 3 to 8 carbon atoms,
- the term "substituted" qualifying "alkyl", "alkoxy", "alkenyl", "alkynyl", "cycloalkyl" or "cycloalkylalkyl" denoting that those groups are substituted by one or more radicals selected from alkyl, alkoxy and halogen;
- the term "substituted" associated with R₅ and with "phenyl", "phenylalkyl" or "diphenylalkyl" denoting that those groups are substituted by one or more radicals selected from alkyl, alkoxy, halogen, hydroxy and trifluoromethyl;
- their enantiomers and diastereoisomers,
- and the addition salts thereof with a pharmaceutically acceptable acid or base.

2. Compound according to claim 1, which is N-[2-(7-methoxy-3-benzoyl-naphth-1-yl)ethyl]acetamide.

3. Compound according to claim 1, which is N-[2-(7-methoxy-3-acetyl-naphth-1-yl)ethyl]acetamide.

4. Compound according to claim 1, which is N-[2-(7-methoxy-3-acetyl-naphth-1-yl)ethyl]cyclopropylcarboxamide.

5. Compound according to claim 1, which is N-[2-(7-methoxy-3-ethyl-naphth-1-yl)ethyl]acetamide.

6. Compound according to claim 1, which is N-[2-(7-methoxy-3-propyl-naphth-1-yl)ethyl]acetamide.

7. Process for the preparation of compounds of formula (I) according to claim 1, which comprises reacting a compound of formula (II): 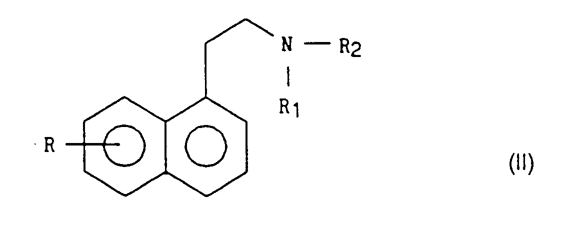 wherein R, R₁ and R₂ are as defined in claim 1,
with a compound of formula (III):
R₆-COCl (III)
wherein R₆ is as defined in claim 1,
to obtain a corresponding compound of formula (Ia): 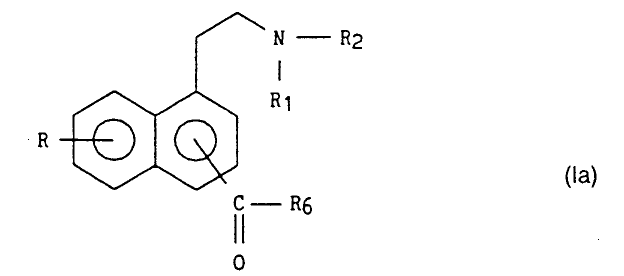 wherein R, R₁, R₂ and R₆ are as defined above,
which may, if desired,
- either be subjected to oxidation by a Baeyer-Villiger reaction to obtain a corresponding compound of formula (Ib): 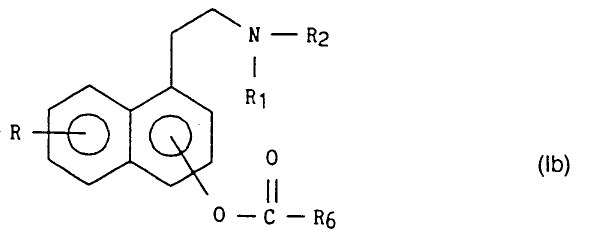 wherein R, R₁, R₂ and R₆ are as defined above, then, if the substituent -O-CO-R₆ is in the 3 position, subjected to hydrolysis in the presence of sodium hydroxide to obtain a corresponding compound of formula (Ic): 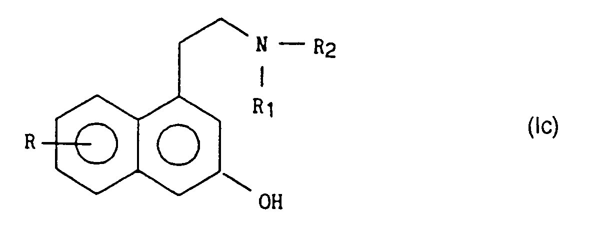 wherein R, R₁ and R₂ are as defined above,
- or be subjected to reduction with mercury and zinc, in the presence of toluene and hydrochloric acid, to obtain a corresponding compound of formula (Id): 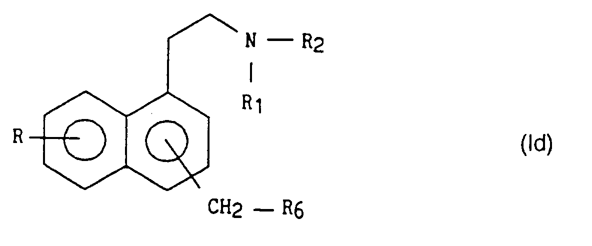 wherein R, R₁, R₂ and R₆ are as defined above,
the compounds of formulae (Ia), (Ib), (Ic) and (Id) forming the totality of the compounds of formula (I),
which compounds of formula (I) may be
- purified according to one or more methods of purification selected from crystallisation, chromatography on silica gel, extraction, filtration and passage over carbon or resin,
- separated, where appropriate, in pure form or in the form of a mixture, into their possible enantiomers or diastereoisomers,
- or converted into salts with a pharmaceutically acceptable acid or base.

8. Pharmaceutical compositions comprising the products of formula (I) according to claim 1 or, where appropriate, an addition salt thereof with a pharmaceutically acceptable acid or base, together with one or more pharmaceutically acceptable excipients.

9. Pharmaceutical compositions according to claim 8 for use in the treatment of melatoninergic disorders.

10. Pharmaceutical compositions according to claim 9 for use in the treatment of seasonal affective disorders, sleep disorders, cardiovascular pathologies, insomnia and fatigue resulting from jet lag, and disorders of the appetite and obesity.

## Patentansprüche

1. Verbindungen der allgemeinen Formel (l): 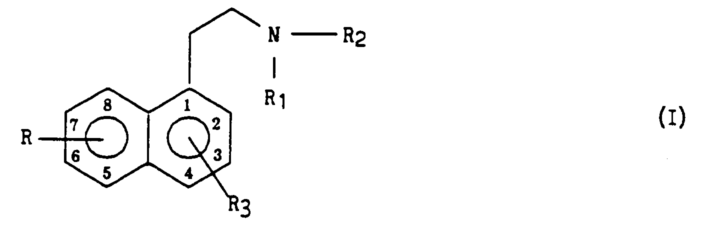 in der:
• R ein Wasserstoffatom oder eine Gruppe ausgewählt aus Alkyl, substituiertem Alkyl und Gruppen -O-R', worin R' ein Wasserstoffatom oder eine Alkylgruppe, substituierte Alkylgruppe, Cycloalkylgruppe, substituierte Cycloalkylgruppe, Cycloalkylalkylgruppe, substituierte Cycloalkylalkylgruppe, Phenylgruppe, substituierte Phenylgruppe, Phenylalkylgruppe, substituierte Phenylalkylgruppe, Diphenylalkylgruppe oder substituierte Diphenylalkylgruppe darstellt;
• R₁ ein Wasserstoffatom oder eine Alkylgruppe;
• R₂
a) 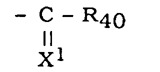 in der X¹ ein Schwefelatom oder ein Sauerstoffatom und R₄₀ ein Wasserstoffatom oder eine Gruppe ausgewählt aus Alkyl, substituiertem Alkyl, Cycloalkyl, substituiertem Cycloalkyl, Cycloalkylalkyl, substituiertem Cycloalkylalkyl, Alkenyl, substituiertem Alkenyl, Alkinyl und substituiertem Alkinyl darstellen;
oder b) 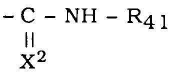 in der X² ein Schwefelatom oder ein Sauerstoffatom und R₄₁ ein Wasserstoffatom oder eine Gruppe ausgewählt aus Alkyl, Cycloalkyl und Cycloalkylalkyl darstellen;
• R₃ eine Gruppe ausgewählt aus:
- (C₂-C₆)-Alkyl,
- substituiertem (C₂-C₆)-Alkyl,
- Cycloalkylalkyl,
- substituiertem Cycloalkylalkyl,
- Alkenyl,
- substituiertem Alkenyl,
- Alkinyl,
- substituiertem Alkinyl,
- Hydroxyl in der 3-Stellung,
- R₅-Alkyl-, worin R₅ substituiert oder nicht substituiert ist und eine Gruppe ausgewählt aus Pyridyl, Phenyl, Naphthyl, Thienyl, Furyl, Pyrimidyl, Indolyl, Benzofuryl, Benzothienyl, Chinolyl und Isochinolyl darstellt;
- R₆-CO-O- und
- R₆-CO-.
R₆ eine Gruppe ausgewählt aus (C₁-C₅)-Alkyl, substituiertem (C₁-C₅)-Alkyl, Cycloalkyl, substituiertem Cycloalkyl, Cycloalkyl-(C₁-C₅)-alkyl, substituiertem Cycloalkyl-(C₁-C₅)-alkyl, R₅- und R₅-Alkyl-, worin R₅, welches substituiert oder nicht substituiert ist, die oben angegebenen Bedeutungen besitzt, darstellt,
bedeuten, mit der Maßgabe, daß
die Verbindung der allgemeinen Formel (I) nicht N-[2-[(4-Acetyl)-naphth-1-yl]-ethyl)-acetamid ist,
- die Begriffe "Alkyl" und "Alkoxy", wenn nichts anderes angegeben ist, geradkettige oder verzweigte Gruppen mit 1 bis 6 Kohlenstoffatomen darstellen,
- die Begriffe "Alkenyl" und "Alkinyl" für ungesättigte, geradkettige oder verzweigte Gruppen mit 2 bis 6 Kohlenstoffatomen stehen,
- der Begriff "Cycloalkyl" für eine cyclische Gruppe mit 3 bis 8 Kohlenstoffatomen steht,
- der Begriff "substituiert" unter Bezugnahme auf "Alkyl", "Alkoxy", "Alkenyl", "Alkinyl", "Cycloalkyl" oder "Cycloalkylalkyl" bedeutet, daß diese Gruppen durch eine oder mehrere Gruppen ausgewählt aus Alkyl, Alkoxy und Halogen substituiert sind;
- der Begriff "substituiert" unter Bezugnahme auf R₅ und "Phenyl", "Phenylalkyl" oder "Diphenylalkyl" bedeutet, daß diese Gruppen durch eine oder mehrere Gruppen ausgewählt aus Alkyl, Alkoxy, Halogen, Hydroxy und Trifluormethyl substituiert sind;
- deren Enantiomere und Diastereoisomere und
- deren Additionssalze mit einer pharmazeutisch annehmbaren Säure oder Base.

2. Verbindung nach Anspruch 1, nämlich N-[2-(7-Methoxy-3-benzoylnaphth-1-yl)-ethyl]-acetamid.

3. Verbindung nach Anspruch 1, nämlich N-[2-(7-Methoxy-3-acetyl-naphth-1-yl)-ethyl]-acetamid.

4. Verbindung nach Anspruch 1, nämlich N-[2-(7-Methoxy-3-acetyl-naphth-1-yl)-ethyl]-cyclopropylcarboxamid.

5. Verbindung nach Anspruch 1, nämlich N-[2-(7-Methoxy-3-ethyl-naphth-1-yl)-ethyl]-acetamid.

6. Verbindung nach Anspruch 1, nämlich N-[2-(7-Methoxy-3-propyl-naphth-1-yl)-ethyl]-acetamid.

7. Verfahren zur Herstellung der Verbindungen der Formel (I) nach Anspruch 1, gemäß dem man eine Verbindung der Formel (II): 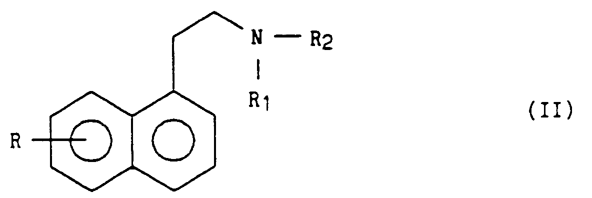 in der R, R₁ und R₂ die in Anspruch 1 angegebenen Bedeutungen besitzen, mit einer Verbindung der Formel (III):
R₆-COCl (III)
in der R₆ die in Anspruch 1 angegebenen Bedeutungen besitzt, umsetzt zur Bildung einer entsprechenden Verbindung der Formel (Ia): 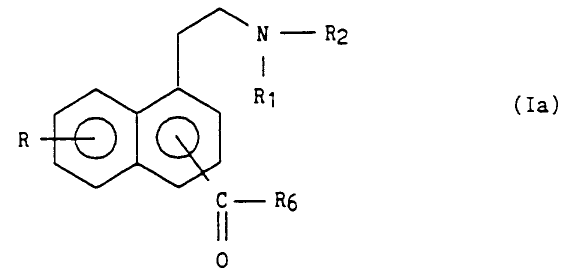 in der R, R₁, R₂ und R₆ die oben angegebenen Bedeutungen besitzen, welche man gewünschtenfalls
- entweder einer Oxidation nach einer Baeyer-Villiger-Reaktion unterwirft zur Bildung einer entsprechenden Verbindung der Formel (Ib): 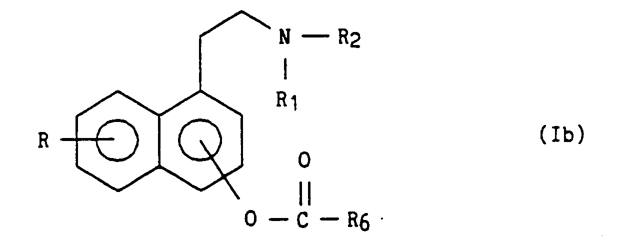 in der R, R₁, R₂ und R₆ die oben angegebenen Bedeutungen besitzen, wonach man, wenn der Substituent -O-CO-R₆ in der 3-Stellung steht, die Verbindung einer Verseifung in Gegenwart von Natriumhydroxid unterwirft zur Bildung der entsprechenden Verbindung der Formel (Ic): 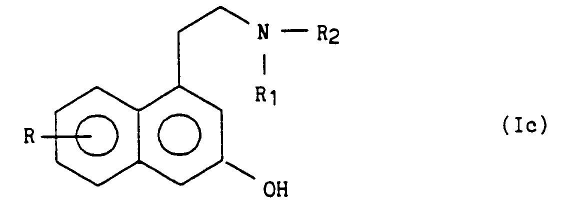 in der R, R₁ und R₂ die oben angegebenen Bedeutungen besitzen,
- oder einer Reduktion mit Quecksilber und Zink in Gegenwart von Toluol und Chlorwasserstoffsäure unterwirft zur Bildung einer entsprechenden Verbindung der Formel (Id): 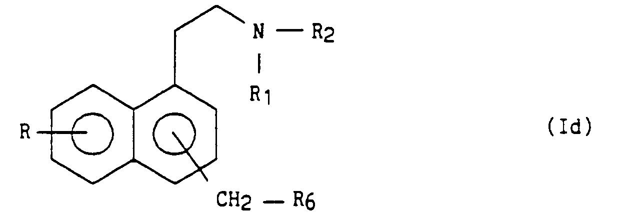 in der R, R₁, R₂ und R₆ die oben angegebenen Bedeutungen besitzen, welche Verbindungen der Formeln (Ia), (Ib), (Ic) und (Id) gemeinsam die Verbindungen der Formel (I) bilden,
welche Verbindungen der Formel (I)
- mit Hilfe eines oder mehrerer Reinigungsverfahren ausgewählt aus Kristallisation, Chromatographie über Kieselgel, Extraktion, Filtration und Überführung über Aktivkohle oder ein Harz gereinigt werden können,
- gegebenenfalls in die entsprechenden Enantiomeren oder Diastereoisomeren in reiner Form oder in Form einer Mischung aufgetrennt werden können
- oder mit Hilfe einer pharmazeutisch annehmbaren Säure oder Base in die Salze überführt werden können.

8. Pharmazeutische Zubereitungen enthaltend die Produkte der Formel (I) nach Anspruch 1 oder gegebenenfalls eines ihrer Additionssalze mit einer pharmazeutisch annehmbaren Säure oder Base in Kombination mit einem oder mehreren pharmazeutisch annehmbaren Trägermaterialien.

9. Pharmazeutische Zubereitungen nach Anspruch 8 zur Behandlung von melatoninergischen Störungen.

10. Pharmazeutische Zubereitungen nach Anspruch 9 zur Behandlung von saisonal bestimmten Depressionen, Schlafstörungen, kardiovaskulären pathologischen Zuständen, der Schlaflosigkeit und der Müdigkeit als Folge von Zeitverschiebungen und Appetitstörungen und der Fettsucht.
